# EUROPEAN PATENT APPLICATION

(11) **EP 3 738 992 A1**
(43) Date of publication of application: **18.11.2020**
(21) Application number: 20162334.5
(22) Date of filing: 06.08.2015
(51) Int. Cl.: C08G 73/02, C08G 63/685, A61K 48/00, C12N 15/87, C08G 83/00

(54) **HYPERBRANCHED POLY(BETA-AMINO ESTER) FOR GENE THERAPY**

(30) Priority: 06.08.2014 GB 201413907
(62) Divisional of application: 15745509.8
(71) Applicant: University College Dublin, National University of Ireland, Dublin, Dublin 4 (IE)
(72) Inventor: WANG, Wenxin, Dublin 4 (IE); ZHOU, Dezhong, Dublin 4 (IE)
(74) Representative: Cooley (UK) LLP

(57) **Abstract**

The invention relates to branched polymers which find use in gene therapy applications as nucleic acid transfection agents. In particular, the invention provides biodegradable, hyperbranched polymers which can be used in gene delivery and which provide improved transfection efficiencies which at the same time are safe and non-toxic.

## Description

### Field of the Invention

The invention relates to branched polymers which find use in gene therapy applications as nucleic acid transfection agents. In particular, the invention provides biodegradable, hyperbranched polymers which can be used in gene delivery and which provide improved transfection efficiencies which at the same time are safe and non-toxic.

### Background to the Invention

Gene therapy promises to be one of the most important frontiers in medicine. Although there have been some major setbacks in the treatment of diseases, many new systems show great potential for effective and safe treatment of genetic related diseases.

The current approaches for gene delivery can be categorized into two groups, viral and non-viral gene delivery. Currently, the majority of gene delivery protocols utilise viral vectors as the gene carriers¹, which are associated with poor control and safety concerns. Non-viral vectors offer a number of advantages over viral systems in that they can be finely tuned and modified to eliminate safety and control issues², however, many of the current non-viral delivery systems lack the transfection and gene releasing capabilities that the viral vectors possess and are much less efficient in delivering the gene to the cell.

Evidence of growth in this technology can be seen from the rise in the number of viral based gene therapy companies (from 44 in 1995 to more than 190 at present).

Non-viral, synthetic linear poly(β-amino esters) for gene delivery are known. However, the limited end groups and linear structure of the linear poly(β-amino esters) severely hinder their further improvement of gene delivery efficiency. Although, non-viral transfection agents overcome many concerns associated with the use of viral vectors, there are typically problems associated with lower transfection efficiency. The advantages of non-viral polymer/liposome transfection reagents are that they consist of simple components, many of which are commercially available and they exhibit fewer biosafety problems. Even though significant improvements have occurred over the past decade, in the field of non-viral gene therapy, the efficiency is still lacking so that many new technologies are unable to move beyond the stage of clinical trials and FDA approval. Furthermore, the translation of gene therapy developments to clinical application has to date been severely restricted by concerns regarding mutagenesis with virus-based delivery systems and efficiency issues with currently available chemical-based reagents.

The present invention utilizes triacrylate monomers which react directly via Michael addition with amine monomers to give hyperbranched poly(β-amino ester) (HPAE). Due to the hyperbranched structure, the multiple end groups and three dimensional (3-D) can be used to further improve the properties of poly(p-amino ester) (HPAE) as non-viral gene delivery vector. The reaction sequence involves random polymerization between amino and acrylate units formed throughout the reaction sequence. The term "hyperbranched" as used herein means a structure generated by the conjugate addition of primary or secondary amines to triacrylates, among which each primary or secondary amine is a conjugate addition to two vinyl groups simultaneously.

Initial DNA complexation, cell viability and cell transfection results show promise for these newly synthesised hyperbranched poly(β-amino ester). Complexes (Polyelectolyte complexes of nucleic acids with polycations) have been investigated as promising delivery vectors for an assortment of therapies. Even though the efficiency of these polymers in cell transfection is not to the level of viral-based vectors, it is also important to note that optimisation and fine tuning of these polymers is very easy. These polymers in combination with the advances in plasmid design will have a profound effect on the non-viral gene therapy field.

This application describes the synthesis and characterisation of hyperbranched poly(β-amino ester) for the effective gene delivery for the treatment of genetic related diseases. The non-viral vectors are synthetic polymers that are synthesised by Michael addition with triacrylate monomers and amine monomers to yield hyperbranched poly(β-amino ester) (HPAE). The HPAE are composed of trimethylolpropane triacrylate (TMTPA), bisphenol A ethoxylate diacrylate (BE), 4-amino-1-butanol (S4) and 3-morpholinopropylamine (MPA). The DNA condensation is achieved by the protonation of tertiary amine under acidic pH value. The hyperbranched structure endows the HPAE multiple synergistic functional groups.

Gene delivery vectors have technical design criteria such as packaging of large DNA plasmids, protection of DNA, serum stability, specific cell targeting, internalisation, endolysosomal escape and nuclear localisation with minimal toxicity to name a few. Other criteria include economic and scale-up production factors: ease of administration, ease of fabrication, inexpensive synthesis, facile purification and safety. Much-studied poly(β-amino ester) for gene delivery is a linear PAE containing tertiary amine groups for complexing DNA. However, most of the cationic based polymers that are currently used in research lack the high efficiency displayed by viral vectors in-vitro and in-vivo. Since cationic polymers seem to work differently with different cell types, our system is flexible enough to allow for small changes to the polymer to accommodate the required clinical target. Recent results showed protein expression in a number of cells using our newly synthesised hyperbranched poly(β-amino ester) (HPAE). This polymer formed complexes with DNA in the nanometre scale (<100nm) which were efficiently taken up by the cell. The excess positive charge of the nano-particles meant that they were capable of escaping the endosome by the "proton sponge" effect ⁷ and degrading in the cytoplasm allowing for release of the DNA and subsequent expression of the protein. The polymers showed higher transfection capability than current gold standard polymers (PEI, sSuperfect, Lipofectamine 2000 and Xfect) with less cytotoxicity and higher DNA condensation capability. HPAE is demonstrated by lowered cytotoxicity and higher transfection efficiency of hela, RDEBK and hADSC.

Patent number: US6998115B2 describes biodegradable poly(βamino esters), which are linear structures based on the monomers 1,6-diaminohexane, N,N-cystaminebisacrylamide and 1,2-diaminoethane. The polymers of the present invention differ from this prior art polymer in that they possess branched structures and are synthesized from different building blocks, The present invention with its monomer combination allow for versatile but controlled synthesis with unique structural properties that can be optimised to reduce cytotoxicity and increase transfection efficiency.

### Object of the Invention

Even though significant improvements have occurred over the past decade in the field of non-viral gene therapy, the efficiency is still lacking with many new technologies unable to move beyond the stage of clinical trials and FDA approval. The object of the present invention is thus to develop a safe and effective gene delivery system for the treatment of diseases, particularly genetic related diseases.

### Summary of the Invention

According to the present invention there is provided a poly-beta amino ester made by:
(a) reacting together to form a polymer P1:
   (i) A triacrylate component having the formula (I) 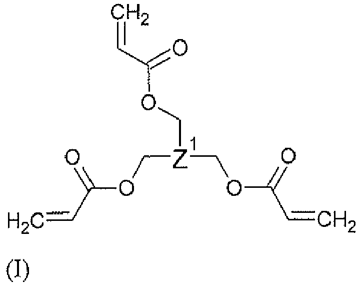 Wherein Z¹ is a scaffold consisting of:
      a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
      wherein Z¹ is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-Cₗo aryl; wherein each R' is independently selected, from the group consisting of hydrogen and C₁-C₆ alkyl;
   (ii) A diacrylate component having the formula (II) 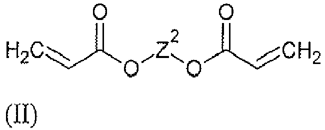
      wherein Z² is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
      wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl; and
   (iii) An amine component A1 comprising 3 to 20 atoms,
      wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl;
      and
(b) reacting the polymer P1 with an amine component A2 comprising 3 to 20 atoms, wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, -OC(O)NR'R', -N(R')C(O)NR'R', -N(R')C(O)OMC₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.

Preferably the poly-beta amino ester has a molecular weight in the range of from about 3000 Da to about 50,000 Da.

The poly-beta amino ester suitably has an alpha parameter derived from the Mark-Houwink equation of less than 0.5. Preferably, the alpha parameter derived from the Mark-Houwink equation is from about 0.2 to about 0.5, more preferably, from about 0.25 to about 0.5, and suitably from about 0.3 to about 0.5.

In one embodiment the poly-beta amino ester suitably has an alpha parameter derived from the Mark-Houwink equation of 0.3 to 0.5.

The triacrylate component may have the formula: 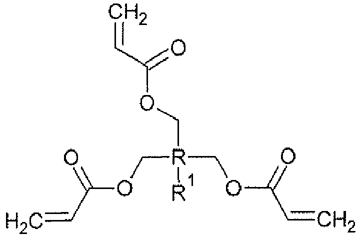
wherein R is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
wherein R is unsubstituted or substituted with at least one of a halogen, a hydroxyl, an amino group, a sulfonyl group, a sulphonamide group, a thiol, a C₁-C₆ alkyl, a C₁C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C_{lO} aryl; wherein each R' is independently selected, from the group consisting of hydrogen and C₁-C₆ alkyl; and
R¹ is an unsubstituted or substituted, linear or branched carbon chain of 1 to 10 carbon atoms, a linear or branched heteroatom-containing carbon chains of 1 to 10 atoms, a carbocycle containing 3 to 10 carbon atoms, or a heterocycle containing 3 to 10 atoms. The triacrylate component may have the formula: 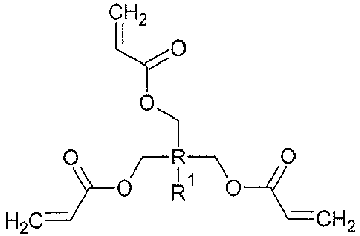 wherein R is a linear or branched carbon chain of 1 to 30 carbon atoms; and R¹ is a linear or branched carbon chain of 1 to 10 carbon atoms.

The triacrylate component may have the formula: 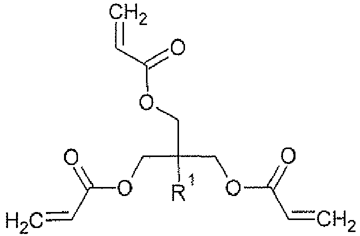 wherein R¹ is a linear or branched carbon chain of 1 to 10 carbon atoms, selected from the group consisting of methyl, ethyl, propyl, butyl, pentyl, hexyl, heptyl, octyl, nonyl or decyl.

Preferably the triacrylate component is selected from the group consisting of trimethylolpropane triacrylate, pentaerythritol triacrylate, glycerol propoxylate (1PO/OH) triacrylate, trimethylolpropane propoxylate triacrylate and pentaerythritol propoxylate triacrylate.

Suitably the diacrylate component has the formula: 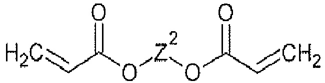
wherein Z² is a linear or branched carbon chain of 1 to 30 carbon atoms or a linear or branched heteroatom-containing carbon chains of 1 to 30 atoms,
wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.

The diacrylate component may have the formula: 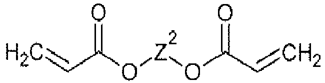
wherein Z² is a linear or branched carbon chain of 1 to 10 carbon atoms,
wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.

The diacrylate component may be selected from the group consisting of 1,3-Butanediol diacrylate, 1,6-Hexanediol diacrylate, Bisphenol A ethoxylate diacrylate, poly(ethylene glycol) diacrylate, 1,4-Butanediol diacrylate and Bisphenol A glycerolate (1 glycerol/phenol) diacrylate.

Preferably the amine component A1 is selected from the group consisting of methyl amine, ethyl amine, propyl amine, butyl amine, pentyl amine, hexyl amine, hetpyl amine, octyl amine, nonyl amine, decylamine.

The amine component A1 may be selected from the group consisting of:

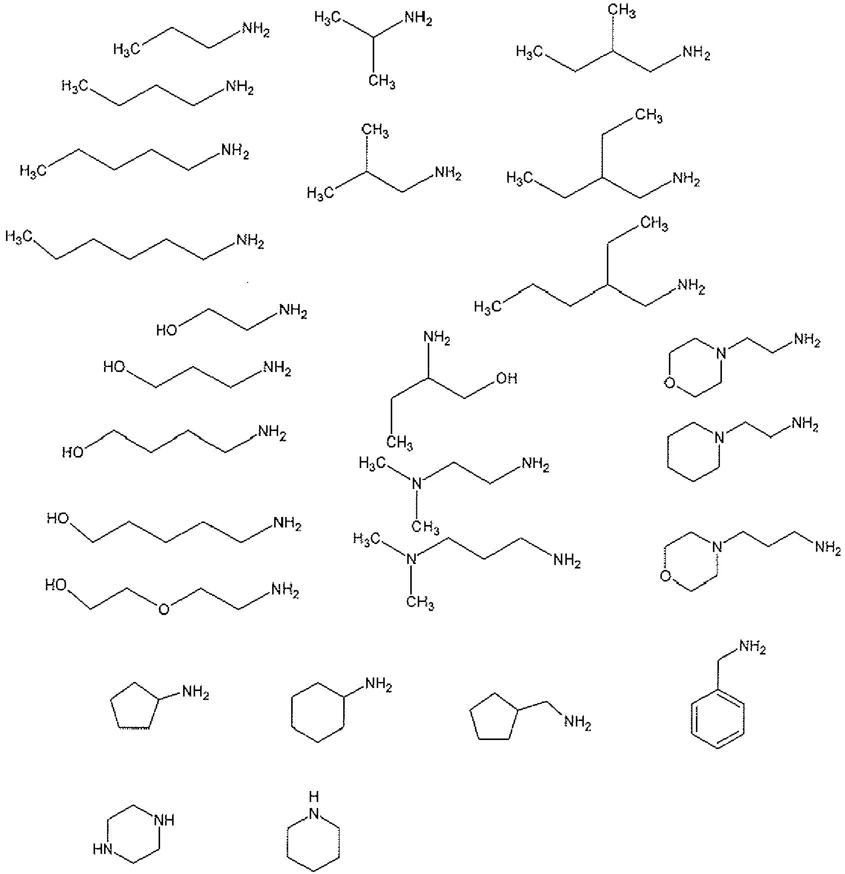

Preferably the amine component A2 is a C1-C20 alkyl amine; a C2-C20 cycloalkyl amine; a C4-C20 aryl amine or a C3-C20 cycloalkyl amine; which can be unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, -OC(O)NR'R', -N(R')C(O)NR'R', -N(R')C(O)OMC₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.

The amine component A2 may be selected from the group consisting of:

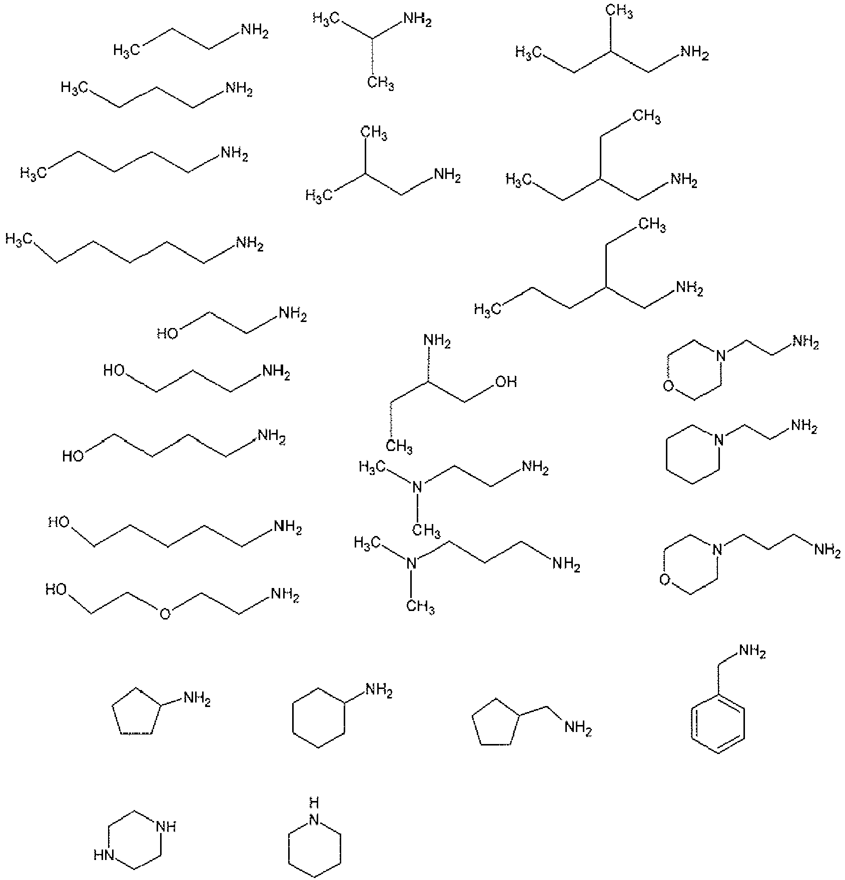

In yet a further embodiment, the present invention provides, a poly-beta amino ester made by:
(a) reacting together, via a Michael addition reaction, to form a polymer P1:
   (i) A triacrylate component having the formula 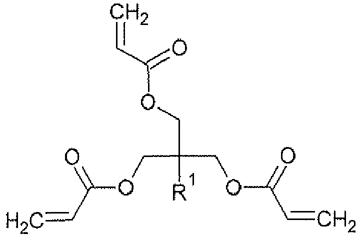
      wherein R¹ is a linear or branched carbon chain of 1 to 10 carbon atoms, selected from the group consisting of methyl, ethyl, propyl, butyl, pentyl, hexyl, heptyl, octyl, nonyl or decyl; or
      wherein the triacrylate component is selected from the group consisting of trimethylolpropane triacrylate, pentaerythritol triacrylate, glycerol propoxylate (1PO/OH) triacrylate, trimethylolpropane propoxylate triacrylate and pentaerythritol propoxylate triacrylate;
   (ii) a diacrylate component selected from the group consisting of 1,3-Butanediol diacrylate, 1,6-Hexanediol diacrylate, Bisphenol A ethoxylate diacrylate, poly(ethylene glycol) diacrylate, 1,4-Butanediol diacrylate and Bisphenol A glycerolate (1 glycerol/phenol) diacrylate; and
   (iii) An amine component A1 comprising 3 to 20 atoms, wherein the amine component A1 is selected from the group consisting of methyl amine, ethyl amine, propyl amine, butyl amine, pentyl amine, hexyl amine, hetpyl amine, octyl amine, nonyl amine, decylamine; or
      wherein the amine component A1 is selected from the group consisting of: 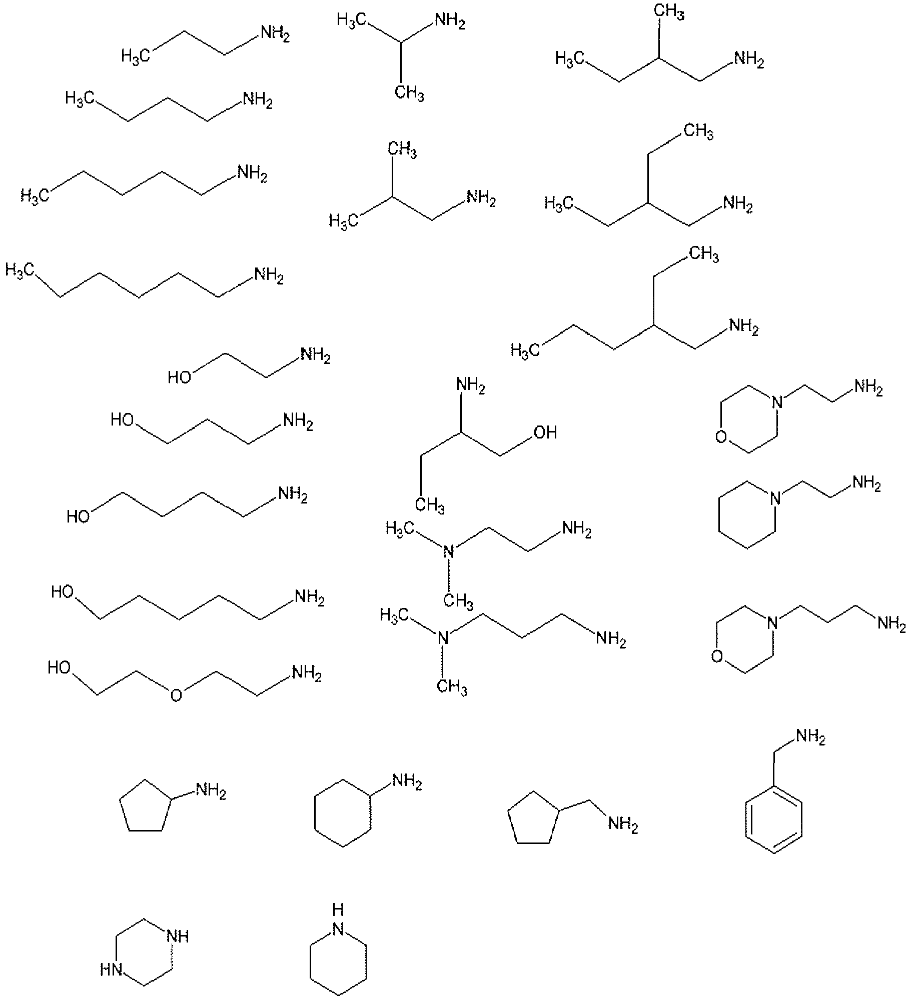 and
(b) reacting the polymer P1 via a Michael addition reaction with an amine component A2 comprising 3 to 20 atoms,
   wherein the amine component A2 is selected from the group consisting of:

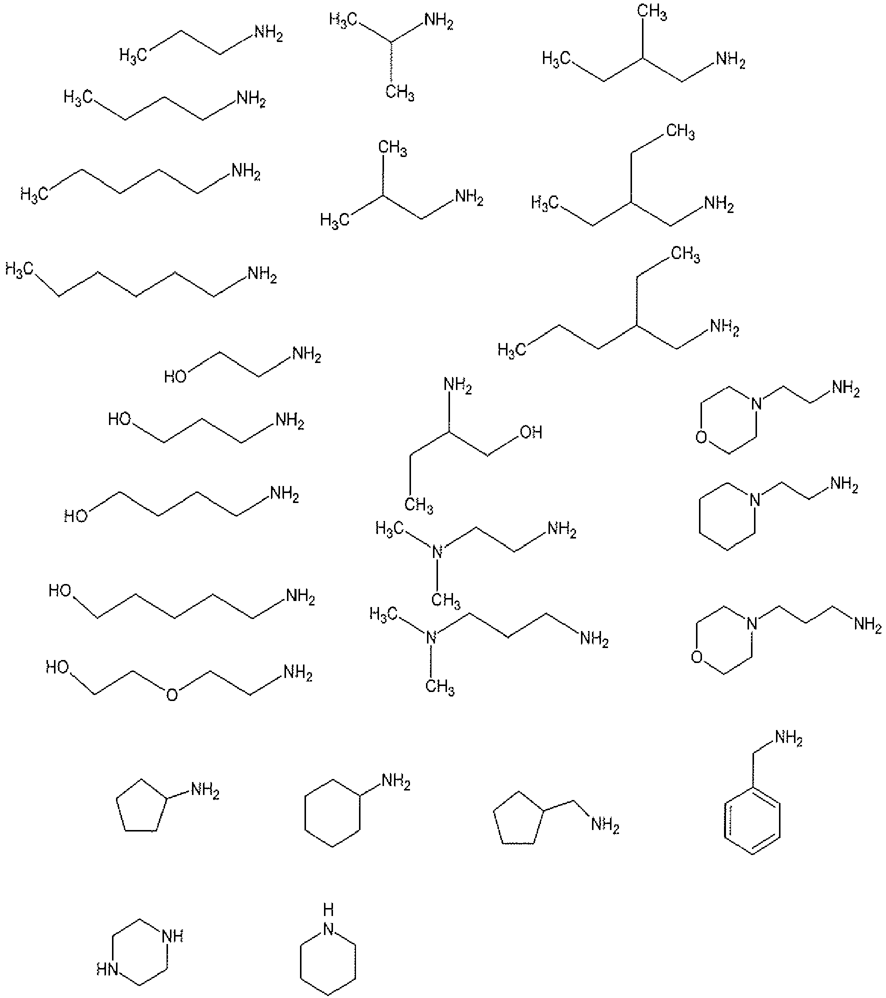

Suitably, the poly-beta amino ester has an alpha parameter derived from the Mark-Houwink equation of less than 0.5, for example, the poly-beta amino ester may have an alpha parameter derived from the Mark-Houwink equation of 0.3 to 0.5.

The invention also provides a poly-beta amino ester as described above for use as a medicament. In a still further aspect the invention provides a pharmaceutical composition comprising a poly-beta amino ester as defined above. The pharmaceutical composition may comprise a polynucleotide and a poly-beta amino ester as defined herein. A pharmaceutical composition comprising nanoparticles containing a polynucleotide and a poly-beta amino ester according to any preceding claim.

### Brief Description of the Drawings

**Figure 1** GPC curve of the synthesised HPAE.
**Figure 2** NMR spectrum of the synthesised HPAE
**Figure 3** Size (A) and charge (B) measurements of complexes formed from various transfection agents as obtained from Zetasizer (n=4).
**Figure 4** Complexation capability of HPAE at different polymer/DNA weight ratios.
**Figure 5** Cell viability studies of HPAE, in Hela cells, RDEBK cells and hADSC cells at optimal polymer/DNA weight ratios. Controls (SF, LP, PEI, Xfect and NFBfect) also have an optimal w/w ratio, (n=4)(±S).
**Figure 6** Luciferase expression quantified by luminescence after transfection with various transfection agents, (n=4)(±S),
**Figure 7****.** Expression of pGFP in transfected HeLa cells (A) and RDEBK cells (B) visualised using a fluorescence microscope.
**Figure 8****.** Amine, triacrylates and diacrylate are firstly copolymerized to form acrylate terminated base polymer followed by end-capping agent of the base polymers to produce HPAE.
**Figure 9** Characterization of HPAEs of varying composition and structure, (a) ¹HNMR spectra of HPAEs and LPAE. (b) GPC traces show that the HPAEs and LPAE have molecular weights around 12,000 Da; (c) Mark-Houwink (MH) plots of HPAEs and LPAE. With the increase in the feed ratio of TMPTA to BE, the α value of LPAE and HPAEs decreases sequentially: LPAE has an α value of 0.65 confirming its typical linear structure. In contrast, the α value of HPAE decreases from 0.48 (HPAE-1) to 0.31 (HPAE-10) proving their highly branched structures.

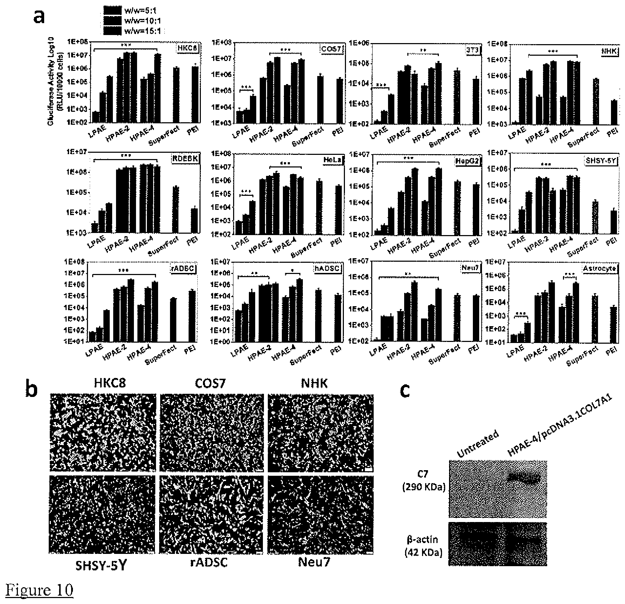

**Figure 10****.** Gene transfection potency of HPAE-2 and HPAE-4 compared with LPAE and commercial transfection reagents SuperFect and PEI in various cell types. (a) Gluciferase activity of various cells 48 hours post transfection. HPAE-2 and HPAE-4 show up to 8,521 fold higher Gluciferase activity than LPAE, SuperFect and PEI. Data are shown as average ± SD, n=4. Statistical significance compared to SuperFect is *p < 0.05, **p < 0.01 and ***p < 0.001; (b) Representative fluorescence images of cells transfected by HPAE at the w/w ratio of 15:1. The scale bars present 200 µm; (c) West blotting results from the supernatant of RDEBK cells. A clearly visible band for collagen VII protein (C7) is seen after transfection with HPAE/DNA.
**Figure 11****.** ¹HNMR spectra of acrylate terminated base polymers of HPAEs and LPAE. Spectra illustrates that prior to end-capping with MPA, at approximately 6.0 ppm, multiple signal peaks are evident which represent the vinyl groups in the base polymers.

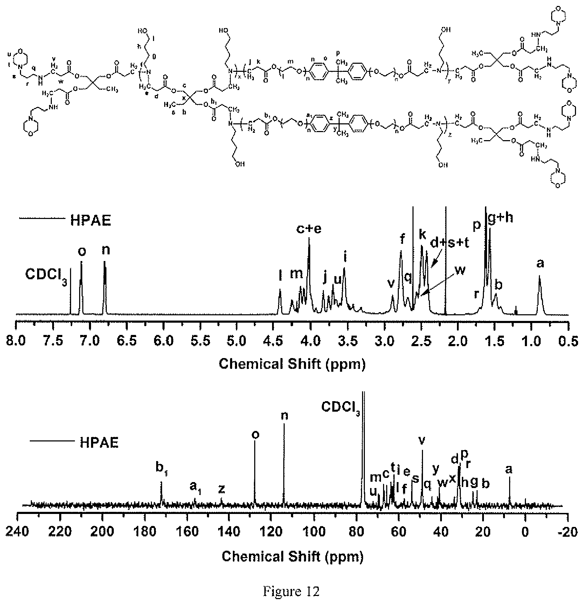

**Figure 12****.** HPAE ¹H NMR and corresponding ¹³C NMR spectrums. Functional groups were assigned to the corresponding signal peaks on the spectums, respectively: ¹H NMR: 0.8 (bs, C***H₃***CH₂-), 1.3-1.7 (m, CH₃C***H**₂***-**, -C(C***H₃***)₂-, -NHCH₂C***H₂***CH₂N- and-NCH₂(C***H₂***)₂CH₂OH), 2.3-2.6 (m, -NCH₂C***H₂***COO-, -COC***H₂***CH₂N-,-NH***CH**₂*CH₂CH₂N-, -COC***H₂***CH₂NH- and -NHCH₂CH₂C***H₂***N(***CH**₂*)₂), 2.7-2.9 (m,-NC***H**₂*(CH₂)₂CH₂OH and -NH***CH**₂*CH₂COO-), 3.2-3.9 (m, -NC***H**₂*COO-, -NCH₂C***H***₂O- and NCH₂(CH₂)₂C***H***₂OH), 4.0-4.5 (m, *-O(*C***H***₂*)*₂O*-,* -COOC***H**₂*C*-* and-NC***H**₂*CH₂COO-), 6.8 (d, -O-C(C***H***)₂CH-), 7.1 (d, -O-C(CH)₂C***H*-**); ¹³C NMR: 7.4, 22.9, 24.7, 31.0, 31.4, 31.9, 33.6, 40.7, 41.7, 44.4, 48.8, 52.8, 53.7, 54.0, 62.4, 63.7, 65.8, 67.3, 69.2, 69.9, 70.3 113.9, 127.7, 143.6, 156.5, 172.0. Measurement conditions: ¹H NMR, CDCl₃, 500 MHz; ¹³C NMR, CDCI3, 150 MHz.

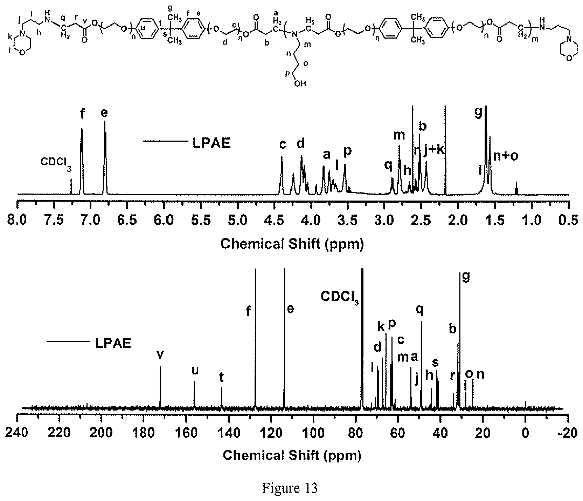

**Figure 13****.** LPAE ¹H NMR and corresponding ¹³C NMR spectrums. Functional groups were assigned to the corresponding signal peaks on the spectrums, respectively: ¹H NMR: 1.5-1.7 (m, -C(C***H₃***)₂-, -NHCH₂C***H**₂*CH₂N- and -NCH₂(C***H**₂*)₂CH₂OH), 2.3-2.6 (m, -COC***H**₂*CH₂N-, -NH***CH**₂*CH₂CH₂N-, -COC***H**₂*CH₂NH- and-NHCH₂CH₂C***H**₂*N(***CH**₂*)₂), 2.7-2.9 (m, -NC***H**₂*(CH₂)₂CH₂0H and -NH***CH**₂*CH₂COO-), 3.4-3.9 (m, -NC***H**₂*COO-, -NC***H**₂*CH₂O- and -NCH₂(CH₂)₂C***H**₂***O**H), 4.0-4.5 (m, - O*(C****H*₂***)*₂O*-),* 6.8 (d, -O-C(C***H***)₂CH-), 7.1 (d, -O-C(CH)₂C***H***-); ¹³C NMR: 24.8, 28.3, 31.0, 31.5, 31.8, 41.0, 41.7, 44.5, 49.3, 53.7, 53.9, 62.6, 65.8, 67.3, 69.2, 69.6, 113,9, 127.7, 143.6, 156.4, 172.3. Measurement conditions: ¹H NMR, CDC13, 500 MHz; ¹³C NMR, CDC13, 150 MHz.
**Figure 14****.** Interaction of HPAE-2 and HPAE-4 with DNA. (a) HPAE-2 and HPAE-4 exhibit strong DNA binding capability and can effectively retard the progression through an agarose gel; (b) HPAE-2/DNA and HPAE-4/DNA polyplexes exhibit very small sizes in DMEM media with 10% FBS; (c) HPAE-4/DNA and HPAE-4/DNA polyplexes exhibit very good stability and no significant aggregation after 4 hours of complex formation in DMEM with 10% FBS.
**Figure 15****.** Preliminary evaluation of transfection efficiency and cytotoxicity of HPAEs of different compositions and structures at a w/w ratio of 10:1 in HeLa cells using Gluciferase assays, (a) All HPAEs exhibit a significant fold increase (12∼2,400) in transfection efficiencies compared to LPAE. Note, HPAE-1, HPAE-2, HPAE-3 and HPAE-4 show 5 to 20 fold increase in transfection efficiency compared to commerical transfection reagents SuperFect and PEI. (b) All HPAEs preserve cell viability above 90%.
**Figure 16****.** GFP positive cells post transfection are quantified by flow cytometry. HPAE-2 and HPAE-4 show significantly enhanced transfection efficiency in various cell types compared to LPAE as well as SuperFect and PEI; Data shown as average ± SD, n=4. Statistical significance compared to SuperFect was *p < 0.05, **p < 0.01 and ***p < 0.001.
**Figure 17****.** Cell viability post transfection with HPAE-2, HPAE-4, LPAE and commercial reagents SuperFect and PEI, measured using Alamarblue assay. Accross 12 different cell types, HPAE-2 and HPAE-4 preserve high levels of cell viability even in stem cells and astrocytes.

### Detailed Description of the Drawings

The process of synthesising these polymers is based on the Michael addition reaction^{1,2}.

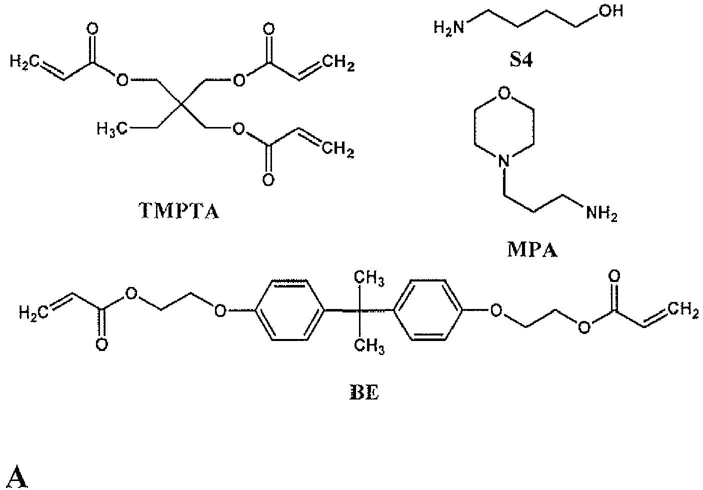

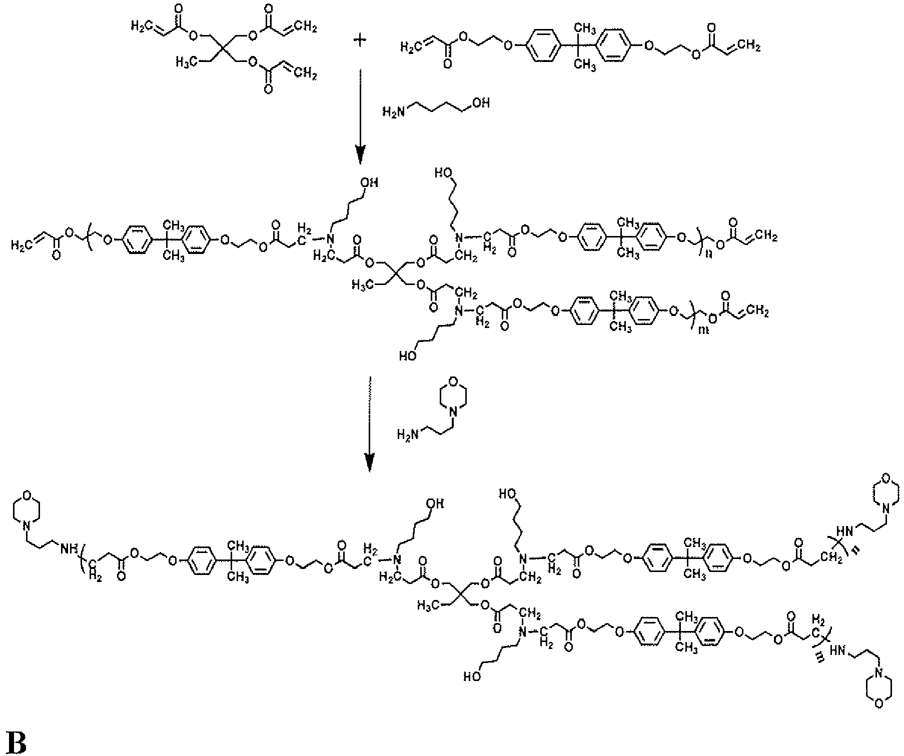

Wherein n and m are any number between 1 and 100, preferably between 1 and 75, preferably between 1 and 50 or preferably between 1 and 25.

The monomers that are used for the synthesis of the HPAE are shown in (A) and the process of HPAE synthesis by Michael addition is shown in (B).

### Experimental Procedure

### Materials.

For polymer synthesis and characterization, commercially available acrylate monomers including trimethylolpropane triacrylate (TMPTA) and bisphenol a ethoxylate diacrylate (BE), amine monomers including 4-amino-1-butanol (S4) and end-capping agents including 3-morpholinopropylamine (MPA) were purchased from Sigma Aldrich and used as received. Lithium bromide (LiBr) for GPC measurements was purchased from Sigma-Aldrich. Solvents dimethyl sulfoxide (DMSO), dimethylformamide (DMF), tetrahydrofuran (THF) and diethyl ether were purchased from Fisher Scientific. Deuterated chloroform (CDCl₃) was purchased from Sigma Aldrich. Sodium acetate (pH 5.2±0.1, 3 M) purchased from Aldrich was diluted to 0.025 M before use. For polyplex characterization and performance, agarose (for gel electrophoresis, Aldrich) and SYBR® Safe Gel Stain (Invitrogen) were used as received according to protocols. For cell culture and gene transfection, cell culture media, trypsin-EDTA and fetal bovine serum (FBS) and Hank's balanced salt solution (HBSS) were purchased from Sigma Aldrich and Life Technologies. The commercial transfection reagents branched polyethyleneimine (PEI, Mw=25 KDa) was purchased from Sigma Aldrich. SuperFect® was purchased from Qiagan, Lipofectamine™ 2000 was purchased from Invitrogen and Xfect™ polymer was purchased from Clontech and used according to manufacturers' protocol. Cell secreted Gaussia princeps luciferase plasmid (pCMV-GLuc) and Green Fluorescent Protein plasmid (pCMV-GFP) were obtained from New England Biolabs UK, and its expansion, isolation and purification was performed using the Giga-Prep (Qiagen) kits as per protocol. BioLux™ Gaussia Luciferase Assay Kit (New England Biolabs), AlamarBlue® (Invitrogen) were used according to protocols. The intercalating agent, propidium iodide was purchased from Life Technologies for flow cytometry analysis. All reagents were used according to the manufacturers' protocols.

### Polymer Synthesis.

To synthesize the hyperbranched PAE (HPAE) polymers, 0.28 g TMPTA, 0.29 g BE and 0.18 g S4 were dissolved in 7.5 ml DMSO, and the reaction occurred at 90°C. Once the molecular weight was in the range of 5000∼7000 Da, 0.288 g MPA dissolved in 2.88 ml DMSO was added to end-cap the acrylate terminated base polymer at RT for 24 h. And then the polymer product was precipitated into diethyl ether three times and dried under vacuum for 24 h and then stored at -20 °C for subsequent studies.

As outlined in Table S1, to synthesize of HPAEs of different compositions and branched structures, various monomer feed ratios were used. Amine, triacrylate and diacrylate were dissolved in DMSO and the reactions were performed at 90 °C. Termination of the polymerization reaction was achieved by end-capping the terminal acrylates via the addition of end-capping amine dissolved in DMSO in the reaction vessel at room temperature (RT) for 24 hours. The final polymer products were purified by precipitating in diethyl ether three times, and then dried under vacuum for 24 h before being stored at -20 °C for subsequent studies. To synthesize the LPAE, amine and diacrylate were directly mixed without solvent and reacted at 90 °C. The reaction was terminated and the final product was purified and stored as described above. Molecular weight (Mw and Mn) and polydispersity index (PDI) of HPAEs and LPAE were measured by gel permeation chromatography (GPC). Analysis was performed using a 1260 Infinite GPC system with a refractive index detector (RI), a viscometer detector (VS DP) and a dual angle light scattering detector (LS 15° and LS 90°). To prepare polymers for analysis, 10.0 mg samples were dissolved in 2 mL DMF and then filtered through a 0.45 µm filter. GPC columns (30 cm PLgel Mixed-C, two in series) were eluted with DMF and 0.1% LiBr at a flow rate of 1 mL/min at 50 °C. Columns were calibrated with linear poly(methyl methacrylate) standards (PMMA). NMR confirmed the compositions of HPAEs. Polymer samples were dissolved in CDCl₃, ¹H NMR spectra were obtained on a Varian Inova 500 MHz spectrometer and reported in parts per million (ppm) relative to the response of the solvent (7.24 ppm) or tetramethylsilane (0.00 ppm). ¹³C NMR spectra were carried out at 150 MHz and reported in ppm relative to the response of the solvent (77.2 ppm).

### Molecular Weight Determination by Gel permeation Chromatography (GPC).

Molecular weight and polydispersity of the HPAE were measured by GPC. A 50 µL HPAE sample was withdrawn from the reaction at specific time intervals, diluted with 1 mL DMF, filtered through a 0.2 µm filter and then analyzed using a Varian 920-LC GPC instrument equipped with a refractive index detector (RI) at 60 °C with DMF as elution solution, the flow rate was 1 ml per min. The machine was calibrated with linear poly(methyl methacrylate) standards.

### Proton Nuclear Magnetic Resonance (¹H-NMR) Measurement.

The chemical structure of HPAE was confirmed by ¹H-NMR. HPAE was dissolved in deuterated chloroform (CDCl₃) and measurements were carried out on a 300 MHz Bruker NMR equipment. All chemical shifts were reported in ppm relative to tetramethylsilane (TMS).

### Polyplex Size and Zeta Potential Determination by Dynamic Light Scattering (DLS).

For size and zeta potential determination, pCMV-GLuc plasmid was used. The polyplexes formed *via* the electrostatic interaction between the DNA and HPAE. Briefly, HPAE was first dissolved in DMSO to 100 mg/ml. 2 µg DNA was diluted in 100 µl sodium acetate buffer (pH 5.2±0.1, 0.025 M). According to the HPAE/DNA weight (w/w) ratio, the required HPAE DMSO solution was diluted to 100 µl with sodium acetate buffer and then added into the DNA solution, vortexed and kept still for 10 minutes. Then polyplex size determination was conducted on a Malvern Instruments Zetasizer (Nano-2590) with scattering angle of 90 °C. For zeta potential determination, the polyplexes were prepared as above and diluted to 800 µl with sodium acetate buffer and then zeta potential determined on the same equipment. All the determinations were repeated at least for four times.

### Determination of the Mark-Houwink alpha parameter

The Mark-Houwink plot is a powerful tool for investigating polymer structure in solution as it clearly reveals the structure-molecular weight relationship with high sensitivity. It is generated by plotting the molecular weight (MW) against the intrinsic viscosity (IV) on a log-log graph. The molecular weight, of course, indicates the length of the polymer chains (or degree of polymerization) but on its own cannot give any indication of structure. The intrinsic viscosity (expressed in dL/g) is a measurement of the molecular density of the polymer chains in solution. The tighter the chains fold or coil in solution, the higher the density and the lower the intrinsic viscosity. This measurement is independent of the molecular weight, so two different structures having the same molecular weight can have different intrinsic viscosities - for example a linear (unbranched) polymer and a branched polymer of the same molecular weight will have different intrinsic viscosities. Furthermore, if the polymer changes structure across its molecular weight distribution (e.g. becomes more substituted), the intrinsic viscosity changes will be easily detected. This is what makes the Mark-Houwink plot so useful and powerful. The raw data for the Mark-Houwink plot is conveniently and simply obtained from high quality multi-detection GPC/SEC data by combining the molecular weight from a light scattering detector with the intrinsic viscosity from a viscometer detector. Both data sets are measured at each point across the elution profile of the sample. The resulting plot can be used in many ways from simply assessing how close two structures are to making complex quantitative measurements of polymer branching. In general:
α<0.5: Compact/spherical chains
0.5<α<0.8: Random-coil/flexible chains
0.5<α<0.8: Rigid-rod/stiff chains

The determination of the Mark-Houwink alpha parameters of the HPAEs was conducted on a 1260 Infinite GPC system with a refractive index detector (RI), a viscometer detector (VS DP) and a dual angle light scattering detector (LS 15° and LS 90°). To prepare polymers for analysis, 10.0 mg samples were dissolved in 2 mL DMF and then filtered through a 0.45 µm filter. GPC columns (30 cm PLgel Mixed-C, two in series) were eluted with DMF and 0.1% LiBr at a flow rate of 1 mL/min at 60 °C. Columns were calibrated with linear poly(methyl methacrylate) standards (PMMA). The GPC data were analysed using universal calibration.

### Agarose Gel Electrophoresis Assays.

For agarose gel electrophoresis assays, 1 µg Gluc plasmid was used for each sample preparation. DNA was diluted to 0.2 µg/µl with sodium acetate buffer, according to w/w ratio, required HPAE was diluted to 5 µl with sodium acetate and then added into the solution of 5 µl DNA, vortexed and kept still for 10 minutes. After that, the polyplex solutions were added along with 2 µl loading dye to the wells in the agarose gel (1% agarose in Tris-boric acid-EDTA (TBE) buffer with SYBR® Safe DNA Stain, pH 8.0) and subjected simultaneously to 60 mV for up to 40 minutes. Then the agarose gel was visualized and imaged with a Vis-Blue™ Transilluminator.

### Cell Culture.

The human-derived renal proximal tubular cell line HKC8, American green monkey kidney fibroblast-like cell line COS7, the Swiss albino mouse embryo tissue cell line 3T3, rat adipose-derived stromal cells rADSC and Neu7 astrocytes, human cervical cancer cell line HeLa (Invitrogen) was cultured in Dulbecco's modified Eagle Medium (DMEM) containing 10% FBS and 1% Penicillin/Streptomycin (P/S). The type VII collagen null-RDEB keratinocytes-RDEB-TA4 cell line RDEBK kindly provided by Dr F, Larcher (Madrid, Spain) was cultured in Keratinocyte Growth Medium 2 (c-20011 pROMOCELL) with 5% FBS and 1% P/S. Human adipose derived mesenchymal stem cell line hADSC (Invitrogen) was maintained in MesenPRO RS™ medium with Basal Medium, Growth Supplement and 1% P/S. The SH-SY5Y neuroblastoma and primary astrocytes were cultured in 50% DMEM / 50% F12 Ham media containing 10% FBS and 1% P/S, the normal human keratinocytes NHK and the recessive dystrophic epidermolysis bullosa keratinocytes RDEBK were cultured in Keratinocyte Growth Medium 2 (c-20011 pROMOCELL) with 1% P/S, the hepatocellular carcinoma cell line HepG2 was cultured in RPMI 1640 media containing 10% FBS and 1% P/S. All the cells were cultured at 37 °C, 5% CO₂, in a humid incubator using standard cell culturing techniques.

### In Vitro Transfection and Cytotoxicity.

For *in vitro* transfections using Gluciferase DNA, cells were seeded on 96-well plates at a density of 1 × 10⁴ cells/well in 100 µL media and cultured until 70-80% confluency. The stem cells rADSC, hADSC used for transfecting were below passage four, while the primary astrocytes and SH-SY5Y used for transfection were under passage five. Prior to the transfection, polyplexes were prepared accordingly, 0.25 µg DNA per well was used for primary cells and stem cells and 0.5 µg DNA per well was used for all other cells. Polymer/DNA weight rations (w/w) of 5:1, 10:1 and 15:1 were used. For commercial transfection reagents, PEI was used at a w/w ratio of 4:1 and SuperFect was used according to the manufacturers' protocol. Briefly, LPAE and HPAEs were initially dissolved in DMSO to 100 mg/mL stock solutions, then according to w/w ratio and finally, the stock solutions were further diluted with sodium acetate buffer. DNA was diluted to 0.1 mg/mL with sodium acetate buffer. LPAE or HPAE solutions were added into the DNA solution, vortexed for 10-15 seconds and allowed to stand for 10-15 minutes. The cell culture media was then added to increase the volume of the polyplex solution to 100 µL. The media in the wells of cell culture plates was removed quickly and the polyplexes solution was added, After 4 hours, the media was replaced with 100 µL fresh media and cells were cultured for another 44 hours. Control cells were subjected to the same treatment minus the addition of polyplexes and comparative controls for both commercial transfection reagents Superfect and PEI were also performed. Analysis of the secreted Gluciferase activity was performed as per the provided protocol, with Gluciferase activity directly detected in the cell supernatant and plotted in terms of relative light units (RLU). Cytotoxicity analysis was performed on all cells using the Almarblue reduction method. To perform this assay, cell supernatants were initially removed and then cells were washed in Hank's balanced salt solution (HBSS) followed by the addition of 10% Alamarblue in HBSS. Living, proliferating cells maintain a reducing environment within the cytosol of the cell which acts to reduce the active, non-fluorescent ingredient (resazurin) in Alamarblue, to the highly fluorescent compound, resorufin. This reduction causes a color change from blue to light red and allows the quantitative measurement of cell viability based on the increase in overall fluorescence and color of the media. The Alamarblue solution from each well was transferred to a fresh flat bottomed 96-well plate for fluorescence measurements at 590 nm. Control cells untreated with polyplexes were used to normalize the fluorescence values, and plotted as 100% viable. All Gluciferase and Alamarblue reduction experiments were performed in quadruplicates with margin of error shown as ± the standard deviation (SD). For *in vitro* transfections using GFP DNA, cells were seeded in 24 well plates at a density of 5 × 10⁴ cells/well in 500 µL media. Transfections were conducted as mentioned above but 1 µg DNA per well was used for primary cells and stem cells while 2 µg DNA per well was used for the other cells. For flow cytometry measurements, after transfection, cells were collected as per standard cell culture protocols and propidium iodide was used to exclude the dead cells with at least 8,000 cells accounted for. To visualize the transfected cells with a fluorescence microscope, 48 hours post transfection, media was removed and cells were washed in HBSS three times and then visualized under the fluorescence microscope (Olympus IX81).

### Evaluation of HPAE/DNA polyplexes.

For agarose gel electrophoresis assays, 1 µg DNA was used for each sample preparation. DNA was diluted to 0.2 µg/µL in sodium acetate buffer (pH 5.2±0.1, 0.025 M). HPAEs were initially dissolved in DMSO to yield 100 mg/mL stock solutions. Based on the w/w ratio, HPAEs were further diluted to 5 µL in sodium acetate buffer and then added into the 5 µL DNA solution, vortexed and allowed to stand for 10 minutes. 2 µL of loading dye was added to each polyplex solution before being loaded into the gel (1% agarose in Tris-Acetate-EDTA buffer with SYBR® Safe DNA Stain, pH 8.3) wells. Gels were run for 40 minutes at 60 mV. Images of agarose gels were acquired with a Vis-Blue™ Transilluminator. To evaluate polyplex size, HPAEs were dissolved in DMSO to result in 100 mg/mL stock solutions. 2 µg DNA was diluted in 10 µL sodium acetate buffer. According to the HPAE/DNA weight ratio (w/w), the HPAE stock solution was diluted to 10 µE with sodium acetate buffer, added into the DNA solution, vortexed for 10 seconds and then allowed to stand for 10 minutes. Polyplexes were diluted with DMEM containing 10% FBS and a Malvern Instruments Zetasizer (Nano-2590) with scattering angle of 90 degree was utilized for polyplex size determination. The size was again determined again after 4 hours of incubation. All experiments were repeated a minimum of four times.

### Western blot assay

RDEBK cells were seeded in a T175 flask 24 hours prior to transfection. HPAE/DNA polyplexes (60 µg DNA) were prepared as mentioned previously and added into the cells. After 48 hours, the supernatant was harvested, concentrated and denatured following standard protocols. The protein solution was loaded into the SDS-PAGE gel (6%) and run at 130 V for 70 minutes followed by 100 V for 15 minutes. Next, the protein sample was transferred onto nitrocellulose membrane at 80 V for 1 hour. Membrane blocking was performed for 1 hour at room temperature using 5% BSA TBST buffer. Incubation of protein sample was performed overnight at 4 °C in primary antibody (collagen VII antibody) diluted in 5% blocking buffer. The membrane was washed three times in TBST (5 minutes each). Following washes, the protein sample was incubated with secondary antibody (anti-rabbit HRP) in 5% blocking buffer for 1 hour at room temperature. Finally, the membrane was washed three times in TBST. Images were gathered using standard darkroom development techniques for chemiluminescence.

### Statistical analysis

All data expressed as average ± SD, with SD represented by error bars. Statistical comparisons between control and treated groups were performed using Student T tests. Average values and standard deviations were calculated for each sample examined from at least four independent experiments. The levels of statistical significance were set at P < 0.05 (*), 0.01 (**) and 0.001 (***).

### Results

### Co-polymer Synthesis:

The hyperbranched poly(β-amino ester) (HPAE) was successfully synthesised using michael addition reaction.

### Complex characterisation:

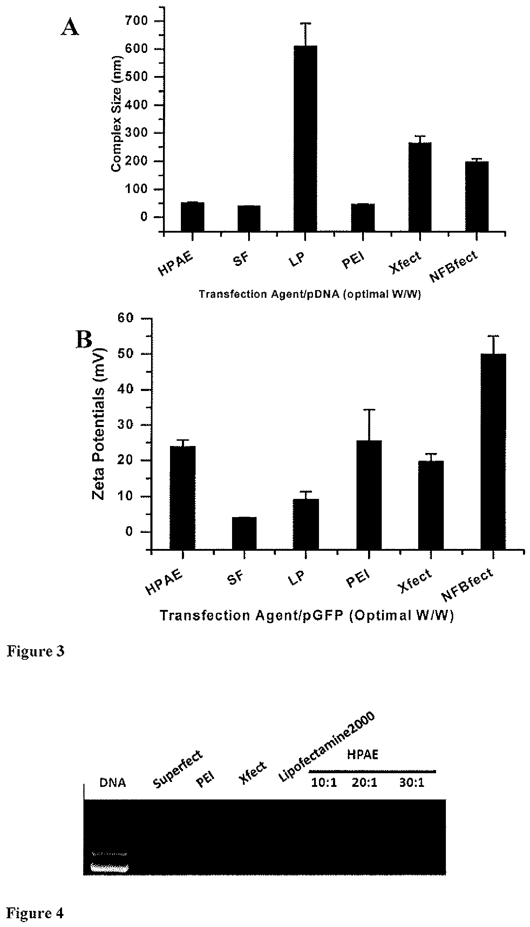

Two of the most significant factors that affect complex uptake by cells are their cationic charge and hydrodynamic size. The ζ potential and hydrodynamic size of the nano-particles were determined using multimode measuring equipment that utilises the DLS and charge properties of colloidal particles to determine the particle size and potential respectively, (figure 3).

### Polymer/DNA complexation:

The ability of the polymer to complex plasmid Gaussia Luciferase (GLuc) DNA was assessed by gel electrophoresis. Polymer/plasmid solutions were made in sodium acetate buffer (pH=5.2, 0.025 M) at various weight ratios by adding 1µg of GLuc to varying concentrations of polymer. These were left for 10 minutes to form complexes before analysis. An agarose gel (1% agarose in Tris-borate-EDTA (TBE) buffer, with SYBR®Safe DNA stain) was made up for all polymers tested. 10µl of each polymer/plasmid solution (DNA concentration of 100 µg ml⁻¹) were added along with 2µl loading dye to each well and subjected simultaneously to 120mV for up to 40 minutes (Fig 4).

### Cell viability:

Cell viability studies for HPAE show lower cytotoxicity in most cell types compared with current commercial polymer vectors.

### Cell Transfection:

Luciferase expression activity analysis of HPAE demonstrated the polymer's capability to efficiently deliver and release DNA into the cell. Gaussia luciferase expression results indicate that HPAE is capable of efficiently transfecting Hela, type VII collagen-null keratinocytes (RDEBK) and human-adipose derived stem cells (ADSCs).

### pGFP expression after transfection

Expression of pGFP in transfected Hela cells (A) and RDEBK cells (B) were visualized using fluorescence microscope.

In conclusion, we have developed well-defined multifunctional HPAE and used as non-viral gene delivery vectors *via* the Michael addition reaction. Systematic investigation of the HPAE synthesized over three different types of cells indicates that the hyperbranched structure plays a critical role in achieving high transfection capability and reduced cytotoxicity. By tailoring the composition and structure, HPAE can achieve ultra-high transfection efficiency and at the same time show very low cytotoxicity, particularly in keratinocyte cell lines. HPAE is much more efficient and safer gene delivery vectors compared to the the commercial transfection reagents Superfect, PEI, Xfect and Lipofectamine 2000. The proof-of-concept HPAE sets a new benchmark in gene delivery capability and can provide first guidelines for the development of high performance non-viral gene delivery vectors.

### Chemical structure analysis by 1H NMR

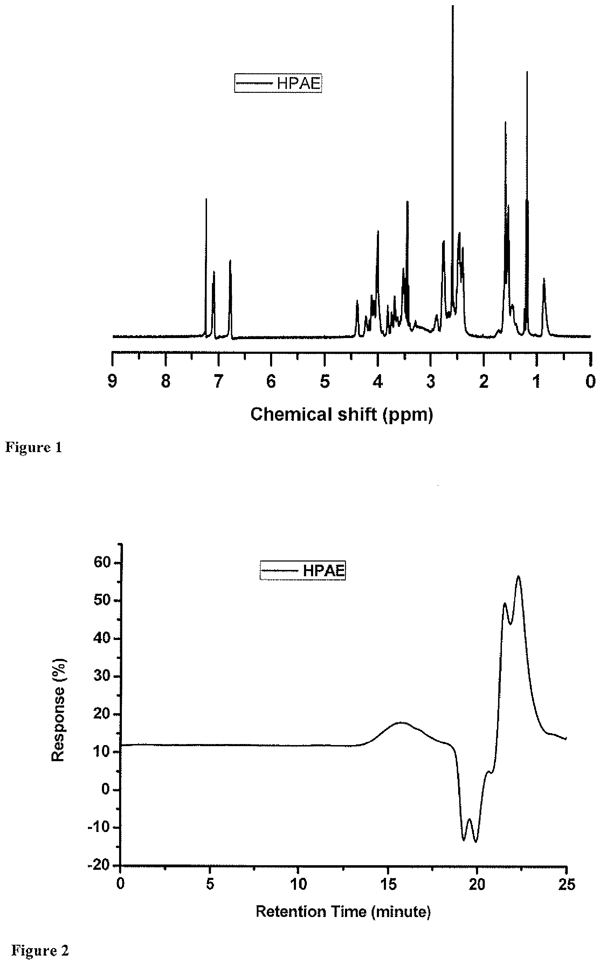

As shown in figure 1, 1H NMR spectrum of HPAE (in CDC13, 300 MHz), the signal peaks of "X" are assigned to residual solvents of DMSO (about 2.5 ppm) and dietheyl ether (about 1.2 ppm and 3.4 ppm).

### Molecular weight measurements by GPC

Figure 2 shows GPC traces of HPAE before and after endcapping with MPA, DMF as elute, 50 degree, 1 ml/min. When conducting the GPC tests to measure the molecular weight of HPAE, DMF was as eluent, the measurements were carried out at 50 degrees, the flow rate of the eluent was 1 ml/min. The molecular weight of HPAE before endcapping with MPA and after endcapping with MPA were measured respectively.

### Size and zeta potential analysis by Dynamic light scattering (DLS)

HPAE/DNA polypexes have hydration diameter about 75 nm and zeta potential about +24 mV, shown in figure 3.

### DNA condensation capacity evaluated by agarose gel electrophoresis

HPAE can condense DNA effectively under w/w ratio ranging from 10:1 to 30:1 (see figure 4).

### Cytotoxicity of HPAE over different cell types evaluated by AlamarBlue assays

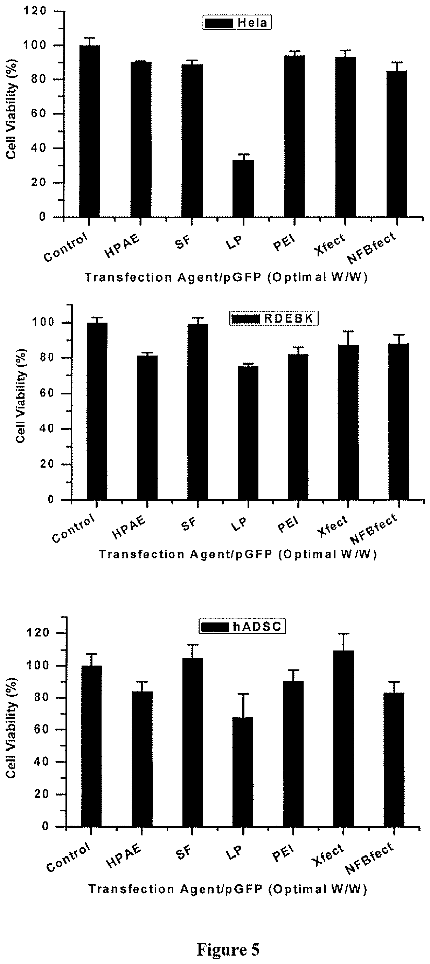

To the three tested cell types, HPAE/DNA can preserve at least 80% cell viability after transfection for 48 h at w/w ratio of 30:1, 96 well plates, 0.5 µg DNA per well (see figure 5).

### In vitro transfection efficiency evaluation with Gluciferase and GFP expression

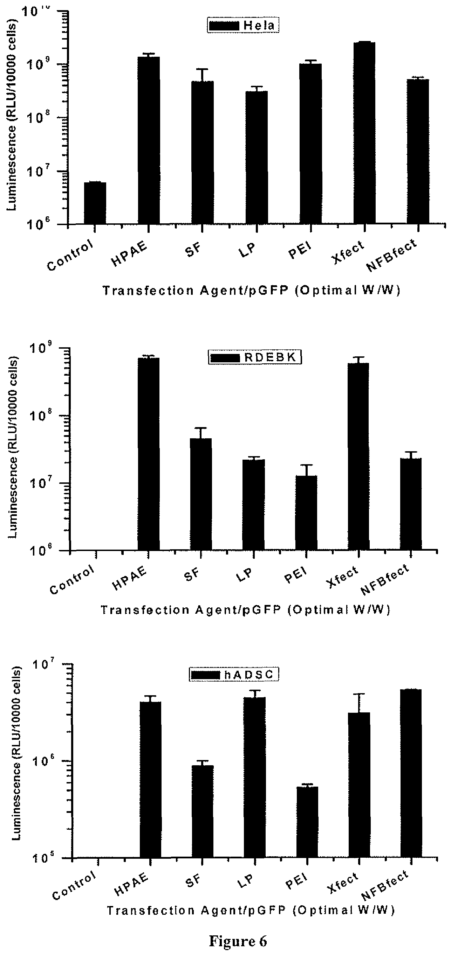

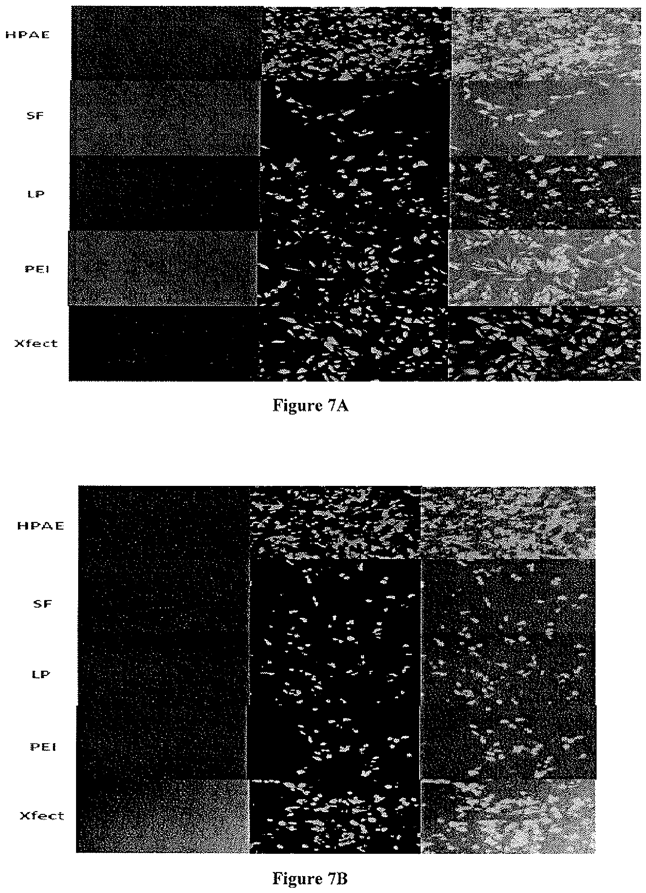

As shown in figure 6 the three cell types exhibited very high Gluciferase activity after transfection with HPAE/DNA polyplexes for 48 h under serum conditions. In addition, as shown in figure 7, the three cell types exhibited very high Green Fluorescent Proteim (GFP) expression after transfected with HPAE/DNA polyplexes for 48 h under serum conditions.

### Design and synthesis of HPAEs

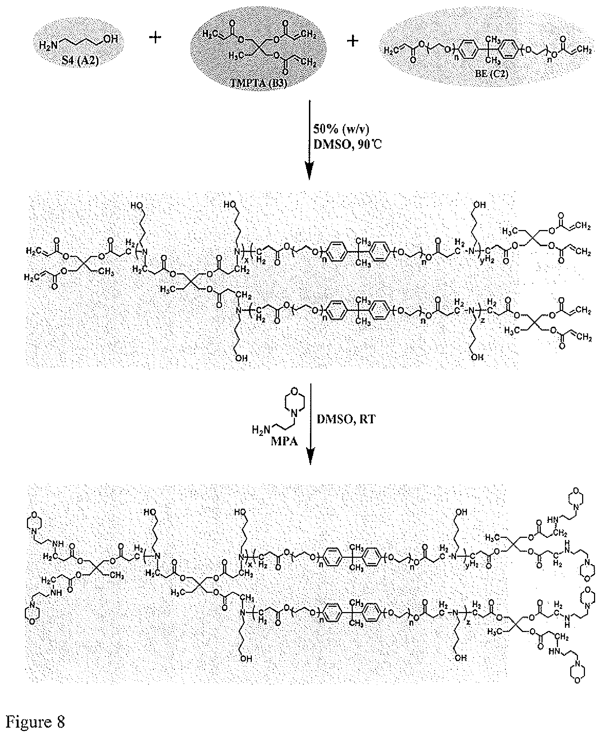

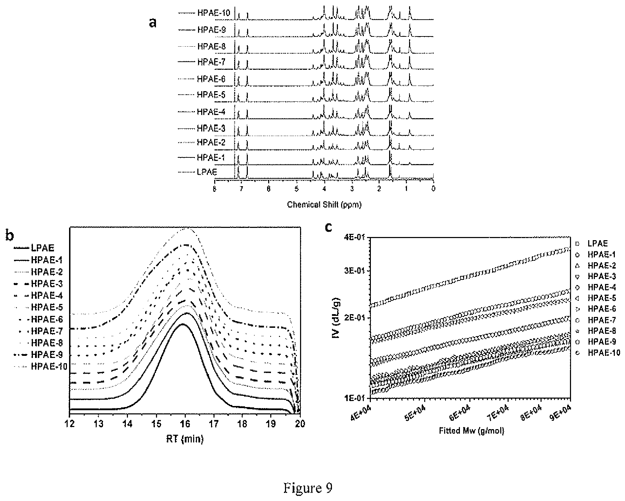

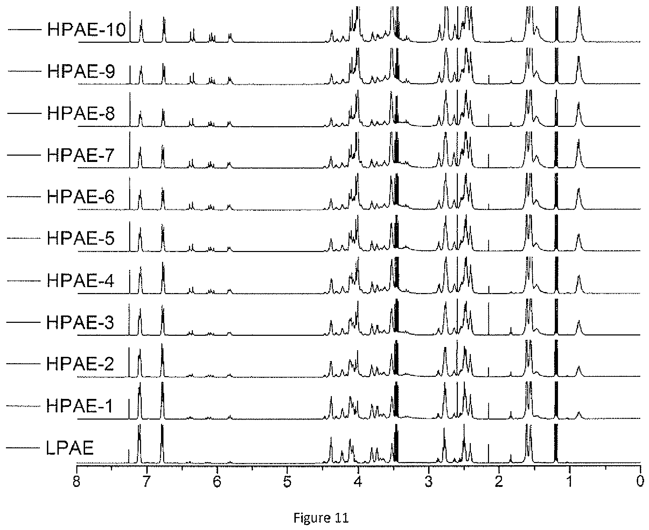

4-amino-1-butanol (S4, A2 type monomer), trimethylolpropane triacrylate (TMPTA, B3 type monomer) and bisphenol A ethoxylate diacrylate (BE, C2 type monomer) were copolymerized via a one-pot "A2+B3+C2" type Michael addition (**Fig. 8**). To systematically assess the effects of different monomer components on the 3D architectures and functionalities of HPAEs, the feed ratios of TMPTA to BE were varied sequentially. Then, functional 3-morpholinopropylamine (MPA) was introduced by end-capping to further enhance the property and functionalities of HPAEs as gene vectors. To compare the effects of different structures on performance of poly(β-amino ester)s, the linear counterpart, LPAE, was also synthesized. Gel permeation chromatography (GPC) was used to monitor the growth of the base polymers during the polymerization. In summary, the increase of the feed ratio of TMPTA to BE resulted in an even faster increase of the molecular weight of the base polymer, which means more oligomers were combined via the branching units. The monomer unit compositions in the base polymers were further verified by nuclear magnetic resonance spectroscopy (¹HNMR and ¹³CNMR, **Fig. 11****,** **12** **and** **13**). The feed ratios indicated that there was an excess of vinyl groups compared to amino groups (**Table S1**), therefore there were multiple unreacted vinyl groups in the base polymers (around 6.0 ppm). As the feed ratio of TMPTA to BE increased, correspondingly the amount of residual vinyl groups in the base polymer increased (**Fig. 11**). The existing multiple vinyl groups were further modified with functional MPA by end-capping. Consistently, after purification, all the vinyl groups in the base polymers were expended and multiple morpholino groups were introduced (**Fig. 9a**). GPC data confirmed that all the HPAEs had a similar molecular weight (Mw was approximately 12, 000, **Fig. 9b**) and PDI (approximately 2.2), which demonstrates that even at high monomer concentration (500 mg/mL) and reaction temperature (90 °C) with a high branching monomer (TMPTA) feed ratio, the polymerization can still be well controlled without gelation via the one-pot "A2+B3+C2" Michael addition approach. The Mark-Houwink (MH) plot alpha (α) value of all the HPAEs was below 0.5 indicating that they were typical highly branched structures (**Fig. 9c**). Furthermore, as the feed ratio of TMPTA to BE increased, the α value decreased from 0.48 for HPAE-1 to 0.31 for HPAE-10 also demonstrating that via the "A2+B3+C2" approach, the branched structure of polymers can easily be adjusted by simply varying the feed ratio of B3 to C2. The detailed monomer feed ratio, polymer composition and structural information are shown in **Table 1.**

**Table 1. Monomer feed ratio, polymer composition and structural information of HPAEs.**

| | **Feed ratio** | | **Composition** | | | |
|---|---|---|---|---|---|---|
| | **[TMPTA]:[BE]^{a}** | **[TMPTA]:[BE]^{b}** | **Mw (Da)^{c}** | **Mn (Da)^{c}** | **PDI^{c}** | **Alpha^{d}** |
| LPAE | 0:1 | 0:1 | 9,771 | 5.820 | 1.6 | 0.65 |
| HPAE-1 | 0.3:1 | 0.4:1 | 10,569 | 4,967 | 2.1 | 0.48 |
| HPAE-2 | 0.6:1 | 0.9:1 | 11,636 | 5,186 | 2.2 | 0.44 |
| HPAE-3 | 0.9:1 | 1.2:1 | 12,264 | 5,449 | 2.2 | 0.41 |
| HPAE-4 | 1.2:1 | 1.5:1 | 11,155 | 4,882 | 2.2 | 0.40 |
| HPAE-5 | 1.5:1 | 1.9:1 | 10,044 | 4,870 | 2.0 | 0.39 |
| HPAE-6 | 1.8:1 | 2.4:1 | 12,497 | 5,044 | 2.4 | 0.36 |
| HPAE-7 | 2.1:1 | 3.1:1 | 9,664 | 4,665 | 2.0 | 0.36 |
| HPAE-8 | 2.4:1 | 3.4:1 | 11,221 | 5,129 | 2.1 | 0.34 |
| HPAE-9 | 2.7:1 | 3.6:1 | 14,278 | 5,519 | 2.5 | 0.33 |
| HPAE-10 | 3:1 | 4.1:1 | 12,426 | 5,341 | 2.3 | 0.31 |

| | | | | | | |
|---|---|---|---|---|---|---|
| a. Reaction condition: DMSO as solvent, 90 °C; Monomer concentration: for LPAE, bulky polymerization, for HPAEs, 500 mg/mL; End-capping: 0.2 mM, RT b. Calculated from ¹H NMR spectra c. Determined by RI detector d. Determined by VS DP detector | | | | | | |

Advantageously, the poly-beta amino esters of the present invention have an alpha parameter derived from the Mark-Houwink equation of less than 0.5, for example of 0.3 to 0.5. This indicates that the triacrylates were copolymerized with the amines and diacrylates simultaneously, the poly-beta amino esters have higher functional group density and three dimensional architecture, and that the poly-beta amino esters of the present invention are highly branched.

**Table S1. Monomer feed ratios for the synthesis of LPAE and HPAEs**

| | **TMPTA:BE** | **TMPTA¹ (µL)** | **BE¹ (µL)** | **S4¹ (µL)** | **MPA (µL)** | **DMSO² (mL)** |
|---|---|---|---|---|---|---|
| LPAE | 0:1 | 0 | 562 | 89 | 146 | 8.0 |
| HPAE-1 | 0.3:1 | 148 | 776 | 178 | 146 | 6.3 |
| HPAE-2 | 0.6:1 | 224 | 590 | 178 | 146 | 5.9 |
| HPAE-3 | 0.9:1 | 272 | 478 | 178 | 146 | 5.6 |
| HPAE-4 | 1.2:1 | 302 | 394 | 178 | 146 | 5.4 |
| HPAE-5 | 1.5:1 | 326 | 346 | 178 | 146 | 5.2 |
| HPAE-6 | 1.8:1 | 346 | 300 | 178 | 146 | 5.1 |
| HPAE-7 | 2.1:1 | 362 | 272 | 178 | 146 | 5.1 |
| HPAE-8 | 2.4:1 | 372 | 244 | 178 | 146 | 5.0 |
| HPAE-9 | 2.7:1 | 380 | 224 | 178 | 146 | 5.0 |
| HPAE-10 | 3:1 | 384 | 206 | 178 | 146 | 4.9 |

| | | | | | | |
|---|---|---|---|---|---|---|
| 1. For the synthesis of HPAE, the monomers: TMPTA, BE and S4 were pre-dissolved in DMSO to 500 mg/mL. 2. Volume of DMSO used to dilute the base polymers prior to end capping with MPA | | | | | | |

### Interaction of HPAEs with DNA

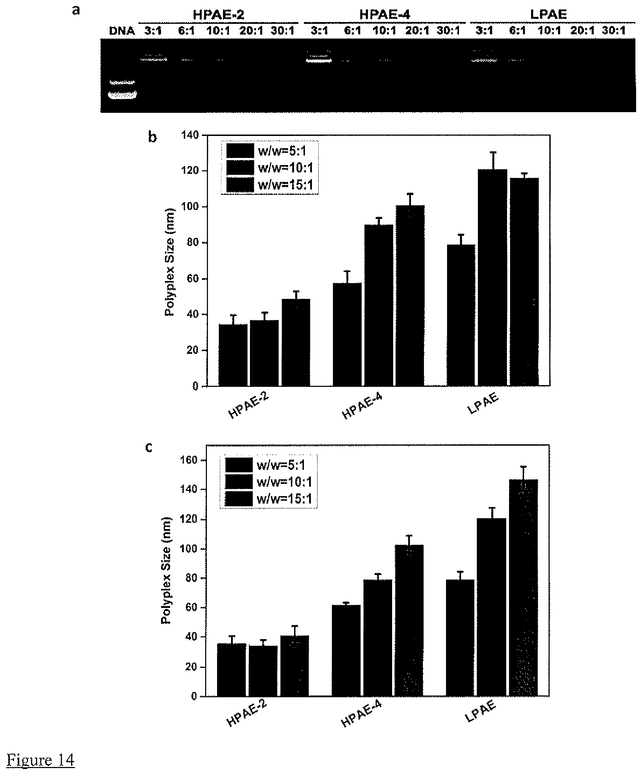

Condensation of DNA by gene delivery vectors into nano sized polyplexes is one of the fundamental requirements for efficient gene delivery. To assess DNA condensation and nano polyplex formation, agarose gel electrophoresis and dynamic light scattering (DLS) assays were used. Gel electrophoresis results demonstrated that there was no DNA shift out of wells across all HPAE/DNA weight ratio (w/w) ranging from 3:1 to 30:1, signifying the formation of HPAE/DNA polyplexes and strong HPAE-DNA binding (**Fig. 14a**), which is due to the excellent protonation ability of the multiple tertiary amines in the HPAE backbones. DLS results further demonstrated the DNA condensation ability of HPAEs to form nano sized polyplexes (30∼120 nm, **Fig. 14b**). The minute size of these polyplexes is highly applicable, as smaller polyplexes could penetrate biological barriers with greater ease. Skin tissue, blood-brain barrier (BBB) and lymph nodes are well-known for being difficult to traverse, highlighting the broad spectrum of targets and applications HPAEs could be exploited in a variety of tissues. Moreover, the HPAE/DNA polyplexes were quite stable because there was no visible aggregation in the presence of serum (**Fig. 14c**), which could potentially increase polyplex uptake into cells and higher transfection efficiency with huge clinical importance for *in vivo* applications.

### Assessment of HPAE transfection potency in vitro

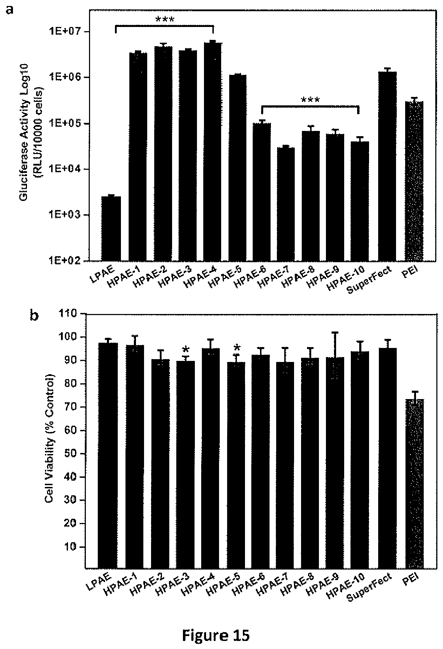

The aim of non-viral gene delivery vector research is predominantly to maximize the transfection efficiency while minimizing the level of cytotoxicity at the same time. However, in practice, improvements in transfection efficiency usually come at the expense of the safety and vice versa. As such, the transfection performance of gene vectors needs to be assessed meticulously prior to final application in a clinic setting. The gene transfection capability and safety of the ten HPAEs with differing compositions and structures was firstly evaluated and compared with that of the LPAE *in vitro* in HeLa cells, and assessed by the secreted Gaussia luciferase (Gluciferase) protein assay and Alamarblue assay. The commercially available transfection reagents SuperFect and PEI were used as positive controls to provide a benchmark for comparison. **Fig. 15** outlines the Gluciferase activity and viability of HeLa cells after transfection with various vectors. The data clearly showed that compared to the LPAE, all ten HPAEs exhibited remarkably higher transfection efficiency with 12 to 2,400 fold enhancement in Gluciferase activity, while maintaining cell viability over 90%. Upon comparison with SuperFect and PEI, which are well established as high transfection efficiency vectors, the Gluciferase activity of HeLa cells after transfection with HPAE-1, HPAE-2, HPAE-3 and HPAE-4 were still substantially 5 to 20 fold higher. These results demonstrate that branching plays a critical role in the transfection potency of poly(β-amino ester)s and that by tailoring the 3D structure along with the multiple functional terminal groups, the transfection efficiency of poly(β-amino ester)s can be enhanced by several orders-of-magnitude.

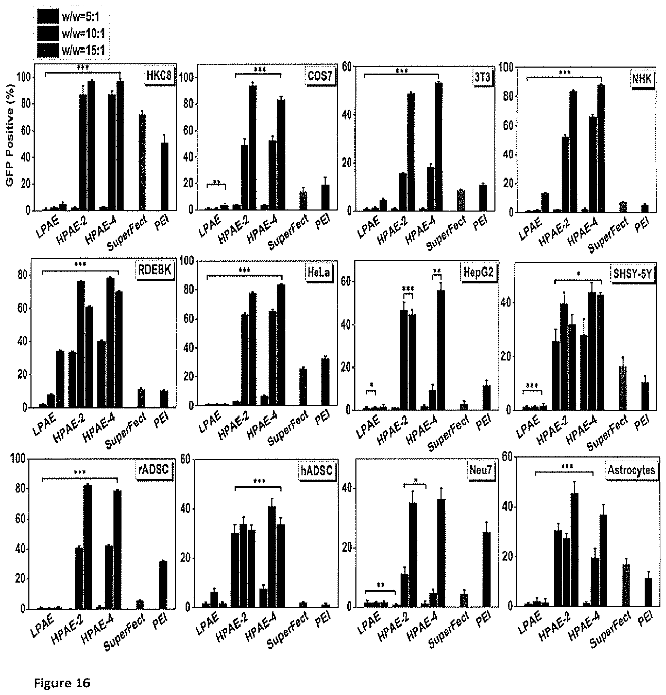

To further verify the high potency of HPAEs in gene delivery, a broad spectrum of 12 cell types with different phenotypes was tested using HPAE-2 and HPAE-4 systematically (**Fig. 10****.** In epithelial cells (HKC8), fibroblasts (COS7 and 3T3), keratinocytes (NHK and RDEBK) and cancer cells (HeLa, HepG2 and SHSY-5Y), both HPAE-2 and HPAE-4 showed superior transfection efficiency, especially at the w/w ratio 15:1. Gluciferase activity was even up to 8,521 fold higher compared to that of the LPAE under the same transfection conditions (**Fig. 10a**). It is well-known that stem cells and astrocytes are among the most challenging cell types to transfect utilizing non-viral vectors. Indeed, on the rat ADSC (rADSC), human ADSC (hADSC), Neu7 astrocytes and primary astrocytes, LPAE exhibited very low transfection efficiency, and in general the Gluciferase activity was 2 to 3 orders-of-magnitude lower than in the above mentioned immortalized cells. In contrast, after transfection with HPAE-2 and HPAE-4 the Gluciferase activity of the same stem cells and astrocytes was much higher and comparable to the immortalized cells, supporting the idea that HPAEs produce high transfection potency regardless of cell types. The superior transfection capability of HPAEs was further quantified using flow cytometry via the expression of GFP after transfection (**Fig. 16**). In the HKC8, COS7, NHK, RDEBK, HeLa and rADSC, over 75% of the cells were GFP positive 48 hours post transfection - even up to 98% in HKC8. Comparatively, less than 10% of cells were GFP positive after transfection by LPAE except in RDEBK (35%) at the w/w ratio of 15:1. Consistent with the Gluciferase expression, even in the most challenging hADSC and primary astrocytes, HPAE-2 and HPAE-4 still showed high transfection efficiency, with over 35% of cells effectively transfected. In a sharp contrast, the LPAE showed negligible transfection efficiency (< 6%) in these cells. Representative fluorescence images of cells after transfection are shown in **Fig. 10b****,** with the superior transfection efficiency of HPAEs demonstrated qualitatively by the strong expression of GFP. High transfection efficiency of HPAEs was further confirmed by a western blotting assay using the pcDNA3.1COL7A1 plasmid coding type VII collagen protein (C7). These results indicated that HPAE-4 can effectively deliver pcDNA3.1COL7A1 into the C7 null RDEBK cells and significant C7 protein was produced 48 hours post transfection (**Fig. 10c**). Taken together, this highlights the ability of HPAEs to deliver a therapeutic gene to restore functional protein expression. All the results from the Gluciferase assays, flow cytometry measurements and western blotting assay substantiate the idea that the introduction of branching can significantly enhance the gene transfection capability of poly(β-amino ester)s, and that branching matters a lot in the gene delivery of poly(β-amino ester)s. It is noteworthy that the twelve cell types transfected here were from various tissues with different phenotypes, so the fact that HPAEs showed superior transfection potency on all of them suggests a great potential for HPAEs with implications for their use across many clinical targets.

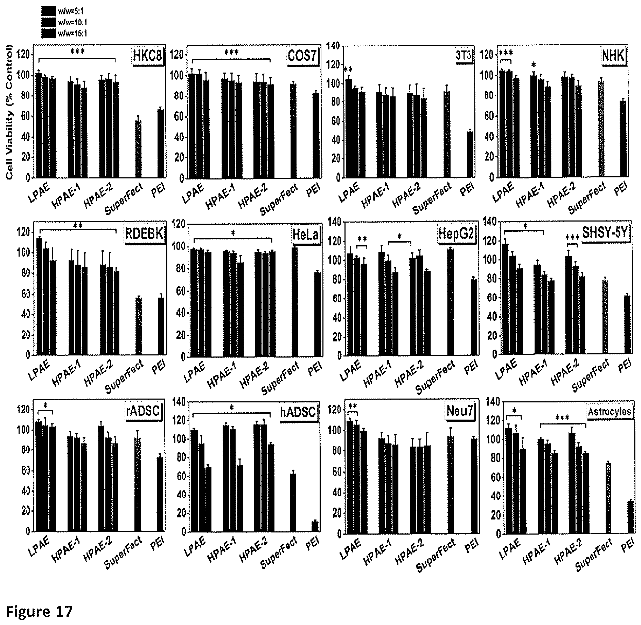

With regards to the transfection safety, **Fig. 17** shows that both HPAEs did not exhibit significant level of cytotoxicities and that they can maintain high cell viability even at high w/w ratio in stem cells and astrocytes. In contrast, the PEI was very toxic and the viability of 3T3, hADSC, SH-SY5Y and primary astrocyte post transfection were all below 50%. These results illustrate that the introduction of branched structure can enhance the transfection capability of poly(β-amino ester)s but does not compromise cell viability.

### References

1. Green, J. J.; Langer, R.; Anderson, D. G., A combinatorial polymer library approach yields insight into nonviral gene delivery. Acc Chem Res 2008, 41(6), 749-59.
2. Eltoukhy, A.A.; Chen, D.; Alabi, C. A.; Langer, R.; Anderson, D. G., Deradable terpolymers with alkyl side chains demonstrate enhanced gene delivery potency and nanoparticle stability. Adv. Mater 2013, 25, 1487-93.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The present invention is further described with reference to the following clauses:
1. A poly-beta amino ester made by:
   (a) reacting together, via a Michael addition reaction, to form a polymer P1:
      (i) A triacrylate component having the formula (I) 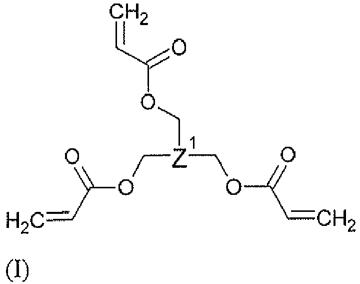 Wherein Z¹ is a scaffold consisting of:
         a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
         wherein Z¹ is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso
            group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C_{lO} aryl; wherein each R' is independently selected, from the group consisting of hydrogen and C₁-C₆ alkyl;
      (ii) A diacrylate component having the formula (II) 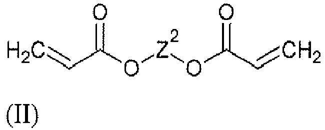
         wherein Z² is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
         wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl; and
      (iii) An amine component A1 comprising 3 to 20 atoms,
         wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl;
         **and**
   (b) reacting the polymer P1 via a Michael addition reaction with an amine component A2 comprising 3 to 20 atoms,
      wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.
2. A poly-beta amino ester according to clause 1 having a molecular weight in the range of from about 3000 Da to about 50,000 Da.
3. A poly-beta amino ester according to any preceding clause, wherein the triacrylate component has the formula: 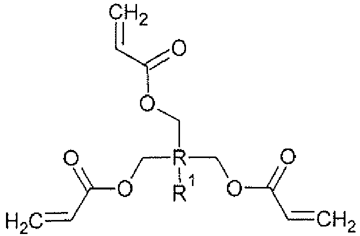
   wherein R is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
   wherein R is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl; and
   R¹ is an unsubstituted or substituted, linear or branched carbon chain of 1 to 10 carbon atoms, a linear or branched heteroatom-containing carbon chains of 1 to 10 atoms, a carbocycle containing 3 to 10 carbon atoms, or a heterocycle containing 3 to 10 atoms.
4. A poly-beta amino ester according to any preceding clause, wherein the triacrylate component has the formula: 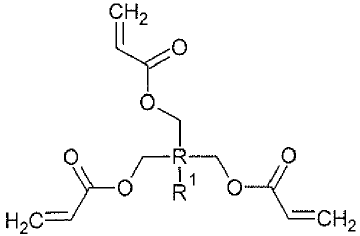 wherein R is a linear or branched carbon chain of 1 to 30 carbon atoms; and R¹ is a linear or branched carbon chain of 1 to 10 carbon atoms.
5. A poly-beta amino ester according to any preceding clause, wherein the triacrylate component has the formula: 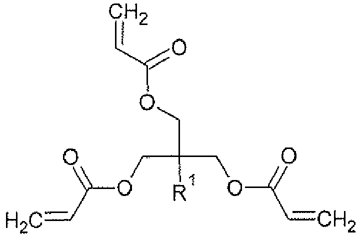 wherein R¹ is a linear or branched carbon chain of 1 to 10 carbon atoms, selected from the group consisting of methyl, ethyl, propyl, butyl, pentyl, hexyl, heptyl, octyl, nonyl or decyl.
6. A poly-beta amino ester according to any preceding clause, wherein the triacrylate component is selected from the group consisting of trimethylolpropane triacrylate, pentaerythritol triacrylate, glycerol propoxylate (1PO/OH) triacrylate, trimethylolpropane propoxylate triacrylate and pentaerythritol propoxylate triacrylate.
7. A poly-beta amino ester according to any preceding clause, wherein the diacrylate component has the formula: 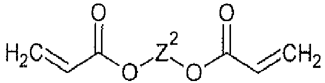
   wherein Z² is a linear or branched carbon chain of 1 to 30 carbon atoms or a linear or branched heteroatom-containing carbon chains of 1 to 30 atoms,
   wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.
8. A poly-beta amino ester according to any preceding clause, wherein the diacrylate component has the formula: 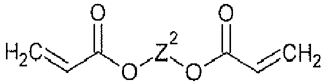
   wherein Z² is a linear or branched carbon chain of 1 to 10 carbon atoms,
   wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.
9. A poly-beta amino ester according to any preceding clause, wherein the diacrylate component is selected from the group consisting of 1,3-Butanediol diacrylate, 1,6-Hexanediol diacrylate, Bisphenol A ethoxylate diacrylate, poly(ethylene glycol) diacrylate, 1,4-Butanediol diacrylate and Bisphenol A glycerolate (1 glycerol/phenol) diacrylate.
10. A poly-beta amino ester according to any preceding clause, wherein the amine component A1 is selected from the group consisting of methyl amine, ethyl amine, propyl amine, butyl amine, pentyl amine, hexyl amine, hetpyl amine, octyl amine, nonyl amine, decylamine.
11. A poly-beta amino ester according to any preceding clause, wherein the amine component A1 is selected from the group consisting of: 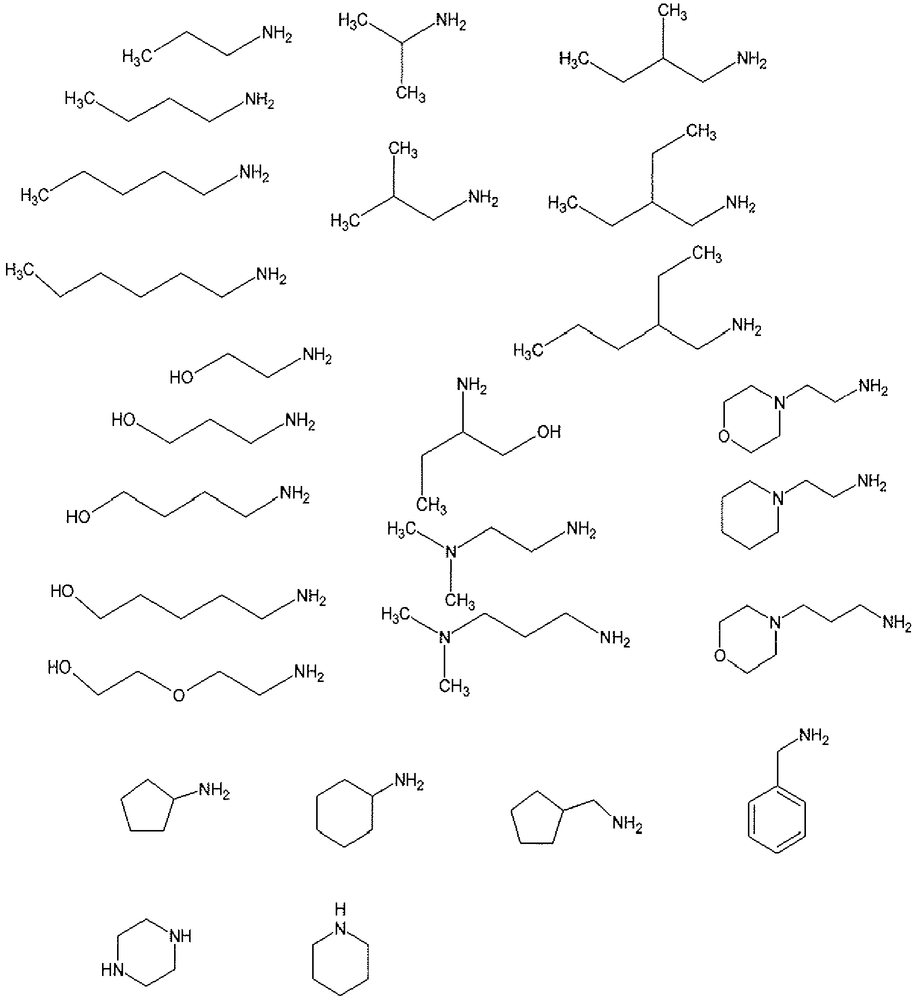
12. A poly-beta amino ester according to any preceding clause, wherein the amine component A2 is a C1-C20 alkyl amine; a C2-C20 cycloalkyl amine; a C4-C20 aryl amine or a C3-C20 cycloalkyl amine; which can be unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.
13. A poly-beta amino ester according to any preceding clause, wherein the amine component A2 is selected from the group consisting of: 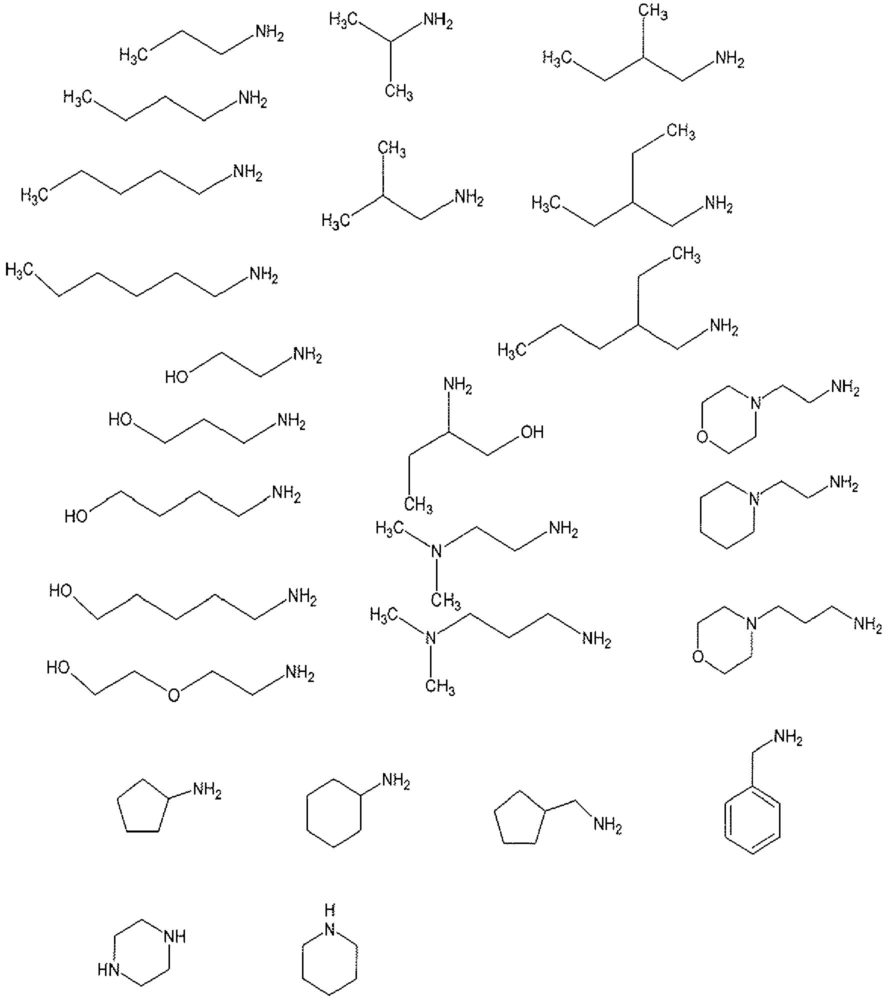
14. A poly-beta amino ester according to any preceding clause for use as a medicament.
15. A pharmaceutical composition comprising a poly-beta amino ester according to any preceding clause.
16. A pharmaceutical composition comprising a polynucleotide and a poly-beta amino ester according to any preceding clause.
17. A pharmaceutical composition comprising nanoparticles containing a polynucleotide and a poly-beta amino ester according to any preceding clause.
18. A composition for transfecting a cell comprising:
   a nucleic acid component and a poly-beta amino ester component,
   wherein the poly-beta amino ester is made by:
      (a) reacting together, via a Michael addition reaction, to form a polymer P1:
         (i) A triacrylate component having the formula (I) 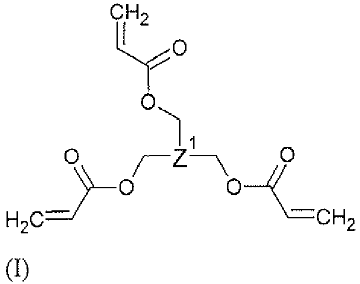 Wherein Z¹ is a scaffold consisting of:
            a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
            wherein Z¹ is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso
               group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl;
         (ii) A diacrylate component having the formula (II) 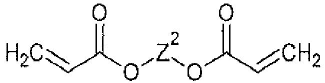 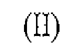
            wherein Z² is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
            wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl; and
         (iii) An amine component A1 comprising 3 to 20 atoms,
            wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl;
            **and**
      (b) reacting the polymer P1 via a Michael addition reaction with an amine component A2 comprising 3 to 20 atoms,
         wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected, from the group consisting of hydrogen and C₁-C₆ alkyl.
19. A method for transfecting cells comprising contacting cells with a composition comprising a non-viral transfection agent and a nucleic acid; wherein said non-viral transfection agent is a poly-beta amino ester made by:
   (a) reacting together via a Michael addition reaction to form a polymer P1:
      (i) a triacrylate component having the formula: 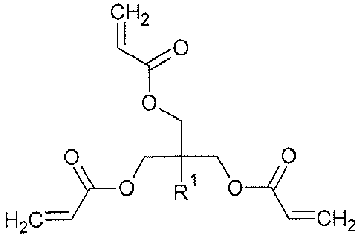 wherein R¹ is a linear or branched carbon chain of 1 to 10 carbon atoms, selected from the group consisting of methyl, ethyl, propyl, butyl, pentyl, hexyl, heptyl, octyl, nonyl or decyl;
      (ii) a diacrylate component selected from the group consisting of 1,3-butanediol diacrylate, 1,6-hexanediol diacrylate, bisphenol A ethoxylate diacrylate, poly(ethylene glycol) diacrylate, 1,4-butanediol diacrylate and bisphenol A glycerolate (1 glycerol/phenol) diacrylate; and
      (iii) an amine component A1 selected from the group consisting of: methyl amine, ethyl amine, propyl amine, butyl amine, pentyl amine, hexyl amine, hetpyl amine, octyl amine, nonyl amine, decylamine; or: 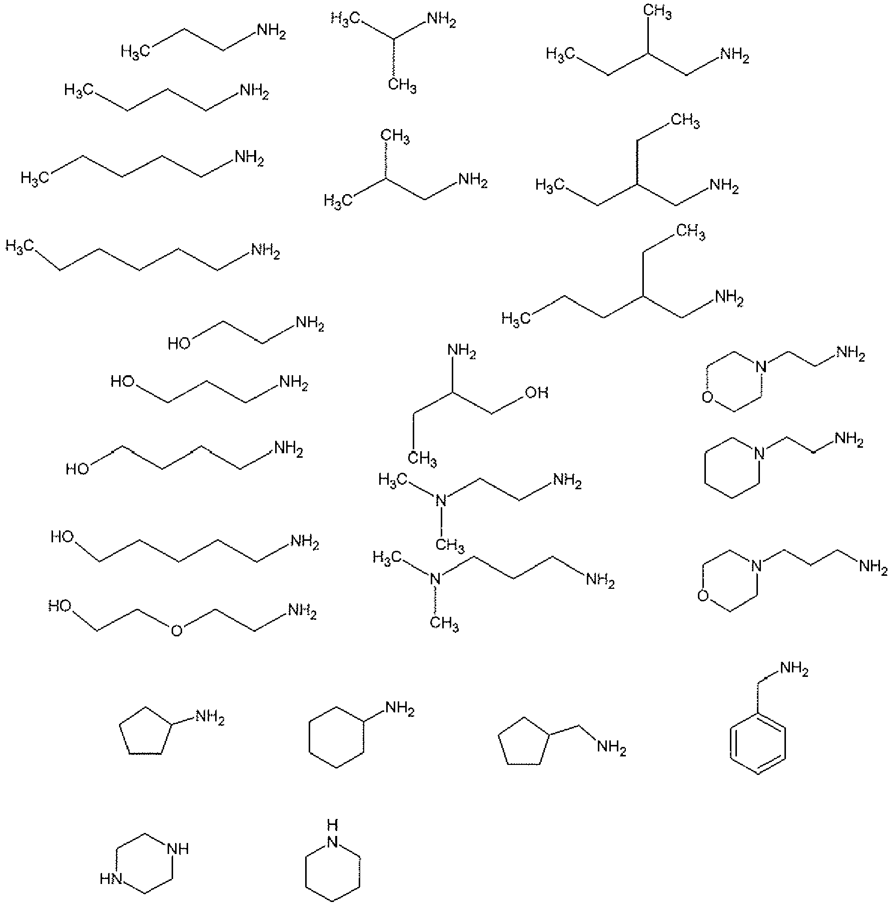 **and**
   (b) reacting the polymer P1 via a Michael addition reaction with an amine component A2; wherein A2 is selected from the group consisting of: 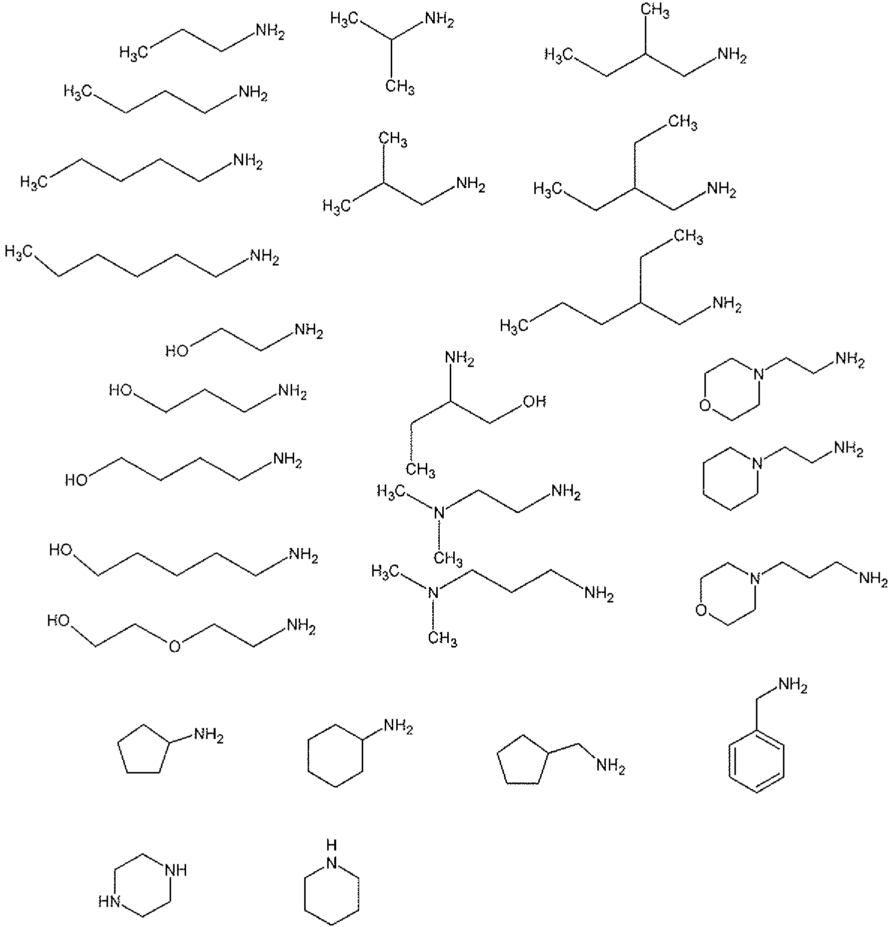
20. The poly-beta amino ester according to any preceding clause having an alpha parameter derived from the Mark-Houwink equation of less than 0.5.
21. The poly-beta amino ester according to any preceding clause having an alpha parameter derived from the Mark-Houwink equation of 0.3 to 0.5.

## Claims

1. A method of making a poly-beta amino ester, comprising:
(a) reacting together, via a Michael addition reaction, to form a polymer P1:
(i) a triacrylate component having the formula (I) 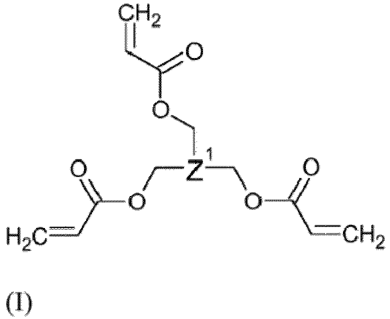 wherein Z¹ is a scaffold consisting of:
a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
wherein Z¹ is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', - N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl;
(ii) a diacrylate component having the formula (II) 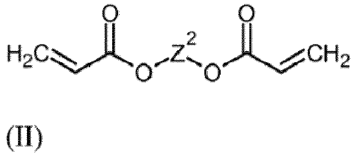 wherein Z² is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms; wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', - N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl; and
(iii) an amine component A1 comprising 3 to 20 atoms,
wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', - N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl;
and
(b) reacting the polymer P1 via a Michael addition reaction with an amine component A2 comprising 3 to 20 atoms,
wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, -OC(O)NR'R', - N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl.

2. A poly-beta amino ester made by:
(a) reacting together, via a Michael addition reaction, to form a polymer P1:
(i) a triacrylate component having the formula (I) 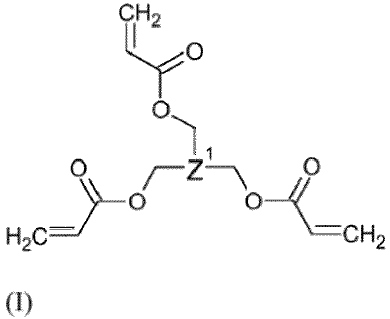 wherein Z¹ is a scaffold consisting of:
a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
wherein Z¹ is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', - N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl;
(ii) a diacrylate component having the formula (II) 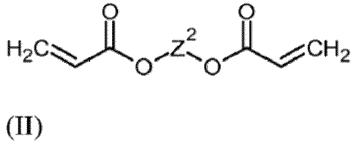
wherein Z² is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R',-N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl; and
(iii) an amine component A1 which is selected from the group consisting of methyl amine, ethyl amine, propyl amine, butyl amine, pentyl amine, hexyl amine, heptyl amine, octyl amine, nonyl amine, decylamine; or
the group consisting of: 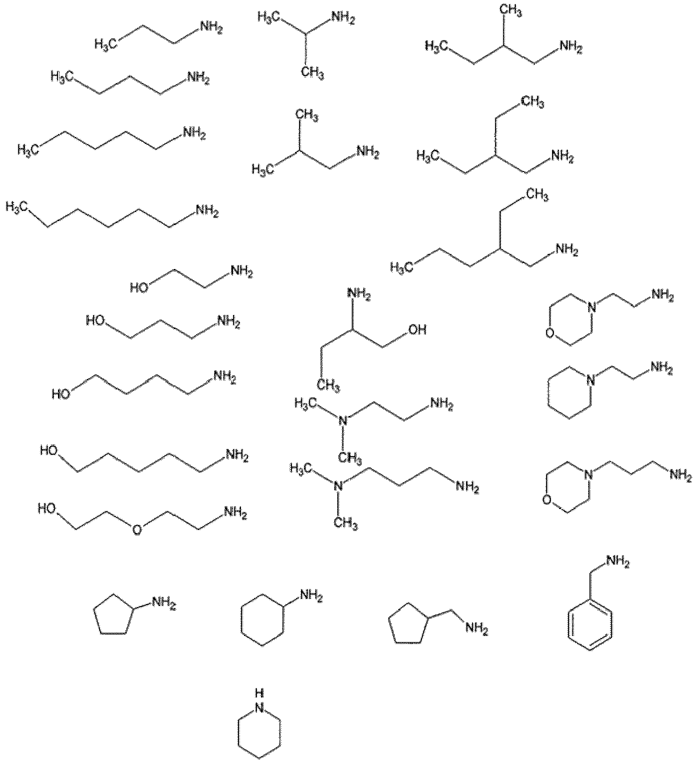 and
(b) reacting the polymer P1 via a Michael addition reaction with an amine component A2 comprising 3 to 20 atoms,
wherein said amine component is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, -OC(O)NR'R', - N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl.

3. The method according to claim 1 or the poly-beta amino ester according to claim 2, wherein the poly-beta amino ester has a molecular weight in the range of from about 3000 Da to about 50,000 Da.

4. The method or poly-beta amino ester according to any preceding claim, wherein the triacrylate component has the formula: 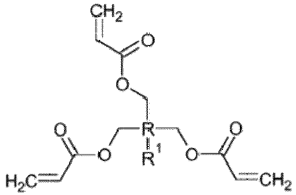
wherein R¹ is a linear or branched carbon chain of 1 to 30 carbon atoms, a linear or branched heteroatom-containing carbon chains of 1 to 30 atoms, a carbocycle containing 3 to 30 carbon atoms, or a heterocycle containing 3 to 30 atoms;
wherein R is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', - N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl; and
R¹ is an unsubstituted or substituted, linear or branched carbon chain of 1 to 10 carbon atoms, a linear or branched heteroatom-containing carbon chains of 1 to 10 atoms, a carbocycle containing 3 to 10 carbon atoms, or a heterocycle containing 3 to 10 atoms.

5. The method or poly-beta amino ester according to any preceding claim, wherein the triacrylate component has the formula: 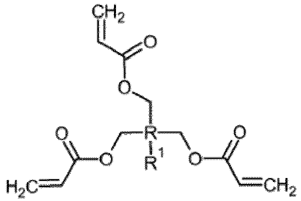 wherein R is a linear or branched carbon chain of 1 to 30 carbon atoms; and R¹ is a linear or branched carbon chain of 1 to 10 carbon atoms.

6. The method or poly-beta amino ester according to any preceding claim, wherein the triacrylate
component has the formula: 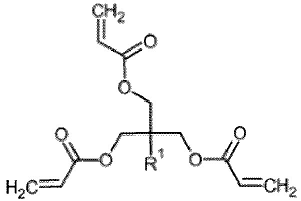 wherein R¹ is a linear or branched carbon chain of 1 to 10 carbon atoms, selected from the group consisting of methyl, ethyl, propyl, butyl, pentyl, hexyl, heptyl, octyl, nonyl or decyl.

7. The method or poly-beta amino ester according to any preceding claim, wherein the triacrylate component is selected from the group consisting of trimethylolpropane triacrylate, pentaerythritol triacrylate, glycerol propoxylate (1PO/OH) triacrylate, trimethylolpropane propoxylate triacrylate and pentaerythritol propoxylate triacrylate.

8. The method or poly-beta amino ester according to any preceding claim, wherein the diacrylate component has the formula: 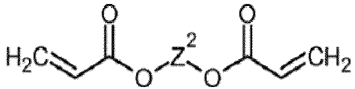
wherein Z² is a linear or branched carbon chain of 1 to 30 carbon atoms or a linear or branched heteroatom-containing carbon chains of 1 to 30 atoms,
wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', - N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl.

9. The method or poly-beta amino ester according to any preceding claim, wherein the diacrylate component has the formula: 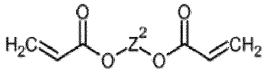
wherein Z² is a linear or branched carbon chain of 1 to 10 carbon atoms,
wherein Z² is unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, a nitroso group, -OC(O)NR'R', -N(R')C(O)NR'R', - N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl.

10. The method or poly-beta amino ester according to any preceding claim, wherein the diacrylate component is selected from the group consisting of 1,3-Butanediol diacrylate, 1,6-Hexanediol diacrylate, Bisphenol A ethoxylate diacrylate, poly(ethylene glycol) diacrylate, 1,4-Butanediol diacrylate and Bisphenol A glycerolate (1 glycerol/phenol) diacrylate.

11. The method according to any preceding claim, wherein the amine component A1 is selected from
(a) the group consisting of methyl amine, ethyl amine, propyl amine, butyl amine, pentyl amine, hexyl amine, heptyl amine, octyl amine, nonyl amine, decylamine, or
(b) the group consisting of:

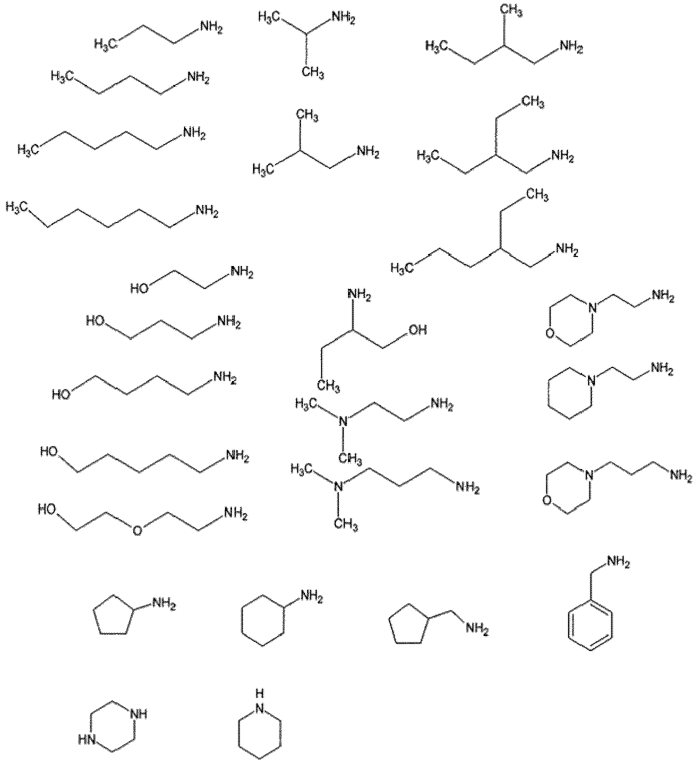

12. The method or poly-beta amino ester according to any preceding claim, wherein
(a) the amine component A2 is a C1-C20 alkyl amine; a C2-C20 cycloalkyl amine; a C4-C20 aryl amine or a C3-C20 cycloalkyl amine; which can be unsubstituted or substituted with at least one of a halogen, a hydroxyl, an amino group, a sulfonyl group, a sulphonamide group, a thiol, a C₁-C₆ alkyl, a C₁-C₆ alkoxy, a C₁-C₆ ether, a C₁-C₆ thioether, a C₁-C₆ sulfone, a C₁-C₆ sulfoxide, a C₁-C₆ primary amide, a C₁-C₆ secondary amide, a halo C₁-C₆ alkyl, a carboxyl group, a cyano group, a nitro group, -OC(O)NR'R', -N(R')C(O)NR'R', -N(R')C(O)O-C₁-C₆ alkyl, C₃-C₆ cycloalkyl, C₃-C₆ heterocyclyl, C₂-C₅ heteroaryl and C₆-C₁₀ aryl; wherein each R' is independently selected from the group consisting of hydrogen and C₁-C₆ alkyl, or
(b) the amine component A2 is selected from the group consisting of:

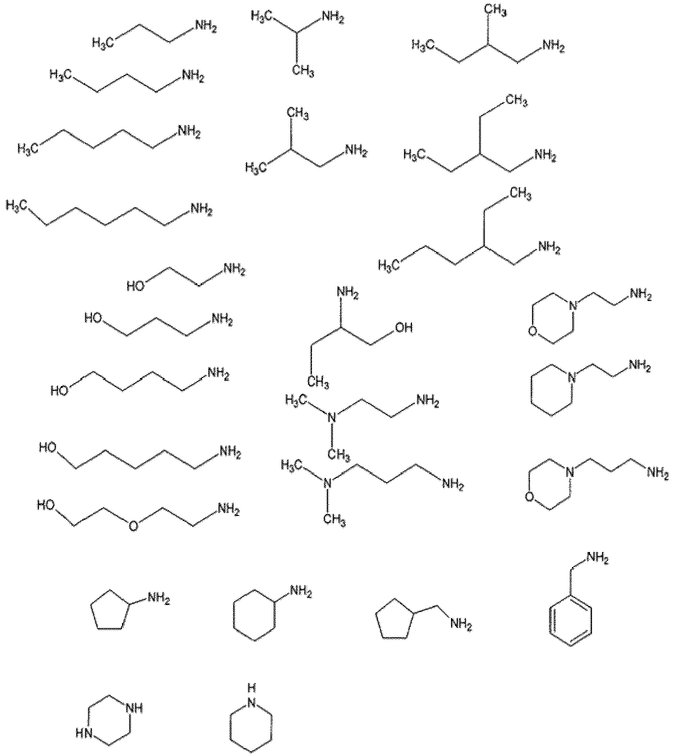

13. The method or poly-beta amino ester according to any preceding claim, wherein the poly-beta amino ester has an alpha parameter derived from the Mark-Houwink equation of less than 0.5, or of 0.3 to 0.5.

14. The poly-beta amino ester according to any one of claims 1-10, 12 or 13; or a poly-beta amino ester made by the method according to any one of claims 1-13; for use as a medicament.

15. A pharmaceutical composition comprising the poly-beta amino ester according to any one of claims 1-10, 12 or 13; or a poly-beta amino ester made by the method according to any one of claims 1-13.

16. The pharmaceutical composition of claim 15, further comprising
(a) a polynucleotide; or
(b) nanoparticles containing a polynucleotide.

17. A composition for use in transfecting a cell, comprising
(i) a nucleic acid component and
(ii) the poly-beta amino ester according to any one of claims 1-10, 12 or 13; or a poly-beta amino ester made by the method according to any one of claims 1-13.

18. An in vitro method for transfecting cells comprising contacting cells with a composition comprising a non-viral transfection agent and a nucleic acid, wherein said non-viral transfection agent is:
(i) the poly-beta amino ester according to any one of claims 1-10, 12 or 13; or
(ii) a poly-beta amino ester made by the method according to any one of claims 1-13.
